# EUROPEAN PATENT APPLICATION

(11) **EP 3 991 747 A1**
(43) Date of publication of application: **04.05.2022**
(21) Application number: 20833655.2
(22) Date of filing: 24.06.2020
(51) Int. Cl.: A61K 39/395, C07K 16/28, A61P 35/00

(54) **PREPARATIONS CONTAINING ANTI-LAG-3 ANTIBODY, AND PREPARATION METHOD THEREFOR AND USE THEREOF**

(30) Priority: 24.06.2019 CN 201910547168
(71) Applicant: Innovent Biologics (Suzhou) Co., Ltd., Suzhou, Jiangsu 215123 (CN)
(72) Inventor: YAO, Tianyi, Suzhou, Jiangsu 215123 (CN); MA, Yidong, Suzhou, Jiangsu 215123 (CN); WANG, Yinjue, Suzhou, Jiangsu 215123 (CN); ZHOU, Kaisong, Suzhou, Jiangsu 215123 (CN)
(74) Representative: Ruscoe, Peter James
(86) International application number: PCT/CN2020/098140
(87) International publication number: WO 2020/259593

(57) **Abstract**

The present invention relates to formulations comprising an anti-LAG-3 antibody, and in particular to pharmaceutical formulations comprising an antibody specifically binding to LAG-3 molecules, a buffer, a stabilizer and a surfactant. Furthermore, the present invention also relates to therapeutic or prophylactic use of these formulations.

## Description

### TECHNICAL FIELD

The present invention relates to the field of antibody formulations. Specifically, the present invention relates to a pharmaceutical formulation comprising an antibody specifically binding to LAG-3 (hereinafter referred to as "anti-LAG-3 antibody"), a method for preparing the pharmaceutical formulation, and therapeutic and/or prophylactic use of the pharmaceutical formulation.

### BACKGROUND

Lymphocyte activation gene 3 (LAG-3), also known as CD223, is a type I transmembrane protein encoded by a LAG-3 gene in the human body. The molecular properties and biological functions of LAG-3 have been well characterized and described, see, e.g., Sierro et al., "The CD4-like molecule LAG-3, biology and therapeutic applications", Expert Opin Ther Targets, 2011, 15 (1): 91-101. LAG-3 is a CD4-like protein expressed on the surface of T cells (particularly activated T cells), natural killer cells, B cells, and plasmacytoid dendritic cells. LAG-3 has been demonstrated to be an inhibitory receptor.

The presence of LAG-3⁺ tumor-infiltrating lymphocytes (TIL) has been reported in patients with melanoma, colon cancer, pancreatic cancer, breast cancer, lung cancer, hematopoietic cancer and head and neck cancer (Demeure C.E. et al., T Lymphocytes infiltrating various tumour types express the MHC class II ligand lymphocyte activation gene-3 (LAG-3): role of LAG-3/MHC class II interactions in cell-cell contacts, Eur. J. Cancer, 2001, 37 (13): 1709-1718). In various cancer mouse models, LAG-3 was blocked by using an anti-LAG-3 antibody, thereby restoring CD8⁺ effector T cells and reducing Treg cell populations.

Anti-LAG-3 antibodies that specifically bind to LAG-3 as LAG-3 blockers are described, for example, in Chinese Patent Application No. 201811561512.X. There is a need in the art for anti-LAG-3 antibody formulations that can be used for treating, preventing or delaying various cancers, immune-related diseases and T cell dysfunctional diseases.

The antibody formulation needs to be formulated in a manner that the antibody is made suitable for administration to a subject and in a manner that the antibody maintains stable in storage and subsequent use as well. For example, if an antibody is not properly formulated in a liquid, the antibody in the liquid solution is prone to decomposition, aggregation, undesirable chemical modification or the like. The stability of an antibody in an antibody formulation depends on the buffer, stabilizer, surfactant and the like used in the formulation.

Although some anti-LAG-3 antibodies are known, there remains a need in the art for novel pharmaceutical formulations comprising anti-LAG-3 antibodies that are sufficiently stable and suitable for administration to a subject. Thus, there is a need for suitable anti-LAG-3 antibody formulations for use in treating or preventing diseases.

### BRIEF SUMMARY

The present invention satisfies the above-described need by providing a pharmaceutical formulation comprising an antibody specifically binding to LAG-3.

In one aspect, the present invention provides a liquid antibody formulation comprising: (i) an anti-LAG-3 antibody, (ii) a buffer, (iii) a stabilizer, and (iv) a surfactant.

The anti-LAG-3 antibody can be any antibody that binds to a LAG-3 molecule (e.g., a human LAG-3 molecule) and blocks LAG-3 signaling, e.g., a polyclonal antibody, a monoclonal antibody, or a combination thereof. Preferably, in one embodiment, the anti-LAG-3 antibody is a monoclonal antibody. In one embodiment, the anti-LAG-3 antibody is a recombinant fully human anti-lymphocyte activation gene 3 (LAG-3) monoclonal antibody disclosed in Chinese Patent Application No. CN201811561512.X filed on Dec. 19, 2018, the content of which is incorporated herein by reference in its entirety for the purpose of the present application. In one embodiment, the anti-LAG-3 antibody comprises a heavy chain variable region and a light chain variable region, wherein the heavy chain variable region comprises a sequence of SEQ ID NO: 7 or a sequence having at least 90% identity thereto, and the light chain variable region comprises a sequence of SEQ ID NO: 8 or a sequence having at least 90% identity thereto:

QLQLQESGPGLVKPSETLSLTCTVSGGSIYSESYYWGWIRQPPGKGLEWIGSIVY
SGYTYYNPSLKSRVTISVDTSKNQFSLKLSSVTAADTAVYYCARVRTWDAAFDI
WGQGTMVTVSS (SEQ ID NO: 7);

DIQMTQSPSSLSASVGDRVTITCQASQDISNYLNWYQQKPGKAPKLLIYDASNL
ETGVPSRFSGSGSGTDFTFTISSLQPEDIATYYCQQVLELPPWTFGGGTKVEIK
(SEQ ID NO: 8).

In one embodiment, the anti-LAG-3 antibody comprises:
- a heavy chain VH CDR1 of GSIYSESYYWG (SEQ ID NO: 1);
- a heavy chain VH CDR2 of SIVYSGYTYYNPSLKS (SEQ ID NO: 2);
- a heavy chain VH CDR3 of ARVRTWDAAFDI (SEQ ID NO: 3);
- a light chain VL CDR1 of QASQDISNYLN (SEQ ID NO: 4);
- a light chain VL CDR2 of DASNLET (SEQ ID NO: 5); and
- a light chain VL CDR3 of QQVLELPPWT (SEQ ID NO: 6).

In one embodiment, the anti-LAG-3 antibody is an IgG4 antibody comprising a heavy chain and a light chain, wherein the heavy chain comprises a sequence of SEQ ID NO: 9 or a sequence having at least 90% identity thereto, and the light chain comprises a sequence of SEQ ID NO: 10 or a sequence having at least 90% identity thereto:

QLQLQESGPGLVKPSETLSLTCTVSGGSIYSESYYWGWIRQPPGKGLEWIGSIVY
SGYTYYNPSLKSRVTISVDTSKNQFSLKLSSVTAADTAVYYCARVRTWDAAFDI
WGQGTMVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEAAGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIA
VEWESNGQPENNYKTTPPVLDSDGSFFLYSRLTVDKSRWQEGNVFSCSVMHEA
LHNHYTQKSLSLSLG (SEQ ID NO: 9)

DIQMTQSPSSLSASVGDRVTITCQASQDISNYLNWYQQKPGKAPKLLIYDASNL
ETGVPSRFSGSGSGTDFTFTISSLQPEDIATYYCQQVLELPPWTFGGGTKVEIKRT
VAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC
(SEQ ID NO: 10).

Preferably, the anti-LAG-3 antibody is the anti-LAG-3 monoclonal antibody ADI-31853 disclosed in Chinese Patent Application No. CN201811561512.X filed on Dec. 19, 2018, which consists of a heavy chain sequence of SEQ ID NO: 9 and a light chain sequence of SEQ ID NO: 10.

In one embodiment, the anti-LAG-3 antibody is an anti-LAG-3 antibody recombinantly expressed in 293 cells or CHO cells.

In one embodiment, the concentration of the anti-LAG-3 antibody in the liquid antibody formulation disclosed herein is about 1-100 mg/mL. In another embodiment, the concentration of the anti-LAG-3 antibody in the liquid antibody formulation disclosed herein is about 5-50 mg/mL. In other embodiments, the concentration of the anti-LAG-3 antibody in the liquid antibody formulation disclosed herein is about 5, 10, 15, 20, 25, 30, 35, 40, 45 or 50 mg/mL.

In one embodiment, the concentration of the buffer in the liquid antibody formulation disclosed herein is about 1-10 mg/mL. In one embodiment, the concentration of the buffer in the liquid antibody formulation disclosed herein is about 4-8 mg/mL, e.g., about 4, 4.5, 5, 5.5, 6, 6.5, 7, 7.5 or 8 mg/mL.

In one embodiment, the buffer is selected from a citrate, a citrate solvate and a combination thereof, and more preferably a citrate or a citrate hydrate, e.g., sodium citrate or sodium citrate dihydrate.

In one embodiment, the concentration of the stabilizer in the liquid antibody formulation disclosed herein is about 10-150 mg/mL. In one embodiment, the concentration of the stabilizer in the liquid antibody formulation disclosed herein is about 15-100 mg/mL, e.g., about 15, 20, 30, 40, 50, 60, 70, 80, 90 or 100 mg/mL.

In one embodiment, the stabilizer is selected from sorbitol, sucrose, trehalose, arginine, arginine hydrochloride and any combination thereof, and preferably sucrose, arginine and/or arginine hydrochloride. In another embodiment, the arginine and/or arginine hydrochloride is present in an amount of about 10-40 mg/mL, preferably about 15-25 mg/mL (e.g., about 15, 16, 17, 18, 19, 20, 21, 22, 23, 24 or 25 mg/mL). In yet another embodiment, the sucrose is present in an amount of about 20-100 mg/mL, preferably about 40-90 mg/mL (e.g., about 40, 50, 60, 70, 80 or 90 mg/mL).

In one embodiment, the concentration of the surfactant in the liquid antibody formulation disclosed herein is about 0.1-1 mg/mL. In one embodiment, the concentration of the surfactant in the liquid antibody formulation disclosed herein is about 0.2-0.8 mg/mL, e.g., about 0.2, 0.3, 0.4, 0.5, 0.6, 0.7 or 0.8 mg/mL.

In one embodiment, the surfactant is a nonionic surfactant. In one embodiment, the surfactant is selected from polysorbate surfactants. In one specific embodiment, the surfactant in the liquid antibody formulation disclosed herein is polysorbate-80.

In some embodiments, the liquid formulation may or may not comprise an edetate (e.g., disodium edetate) and/or methionine.

In one embodiment, the pH of the liquid formulation is about 5.5-7.5. In some embodiments, the pH of the liquid formulation is any of about 5.5-7.5, e.g., about 5.5, 6.0, 6.5, 7.0 or 7.5.

In one embodiment, the liquid formulation is a pharmaceutical formulation, preferably an injection, and more preferably a subcutaneous injection or an intravenous injection. In one embodiment, the liquid formulation is an intravenous infusion.

In one embodiment, the liquid antibody formulation disclosed herein comprises:
(i) the anti-LAG-3 antibody at about 1-100 mg/mL;
(ii) sodium citrate or sodium citrate dihydrate at about 1-10 mg/mL;
(iii) sucrose, arginine and/or arginine hydrochloride at about 10-150 mg/mL; and
(iv) polysorbate 80 at about 0.1-1 mg/mL; wherein
optionally, the liquid formulation does not comprise an edetate (e.g., disodium edetate) and methionine;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 10-30 mg/mL;
(ii) sodium citrate or sodium citrate dihydrate at about 2-8 mg/mL;
(iii) arginine and/or arginine hydrochloride at about 10-40 mg/mL, and/or sucrose at about 40-90 mg/mL; and
(iv) polysorbate 80 at about 0.2-0.8 mg/mL; wherein
optionally, the liquid formulation does not comprise an edetate (e.g., disodium edetate) and methionine;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 20 mg/mL;
(ii) sodium citrate dihydrate at about 5.88 mg/mL;
(iii) sucrose at about 80 mg/mL; and
(iv) polysorbate 80 at about 0.3 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 20 mg/mL;
(ii) sodium citrate dihydrate at about 5.88 mg/mL;
(iii) arginine at about 17.42 mg/mL; and
(iv) polysorbate 80 at about 0.3 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 20 mg/mL;
(ii) sodium citrate dihydrate at about 5.88 mg/mL;
(iii) arginine hydrochloride at about 21.07 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 20 mg/mL;
(ii) sodium citrate dihydrate at about 5.88 mg/mL;
(iii) sucrose at about 50 mg/mL and arginine hydrochloride at about 21.07 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

In one preferred embodiment, the liquid antibody formulation disclosed herein comprises:
(i) the anti-LAG-3 antibody at about 20 mg/mL;
(ii) sodium citrate dihydrate at about 5.88 mg/mL;
(iii) sucrose at about 50 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

In another aspect, the present invention provides a solid antibody formulation obtained by solidifying the liquid antibody formulation disclosed herein. The solidification treatment is implemented by, e.g., crystallization, spray drying, or freeze drying. In one preferred embodiment, the solid antibody formulation is, e.g., in the form of lyophilized powder for injection. The solid antibody formulation can be reconstituted in a suitable vehicle prior to use to give the reconstituted formulation disclosed herein. The reconstituted formulation is also a liquid antibody formulation disclosed herein. In one embodiment, the suitable vehicle is selected from water for injection, organic solvents for injection (including but not limited to, oil for injection, ethanol, propylene glycol, and the like), and combinations thereof.

The liquid formulation disclosed herein can be stably stored for a long period of time, e.g., at least 24 months or longer. In one embodiment, the liquid formulation disclosed herein can be stable after storage at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C, for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the liquid formulation disclosed herein can be stably stored for at least 24 months. In another embodiment, the liquid formulation disclosed herein is stable at a temperature of at least 40 °C. In yet another embodiment, the liquid formulation disclosed herein remains stable at about 2-8 °C for at least 12 months, preferably at least 24 months. In one embodiment, the liquid formulation disclosed herein remains stable at room temperature or, e.g., about 25 °C, for at least 3 months, preferably at least 6 months. In another embodiment, the liquid formulation disclosed herein remains stable at about 40 °C for at least 1 month. In yet another embodiment, the liquid formulation disclosed herein remains stable with shaking at about 5-40 °C, e.g., 25 °C, for at least 1 day, e.g., 3 days or 5 days.

In one embodiment, the stability of the formulation can be indicated after storage by detecting changes in the appearance, visible particles, protein content, purity and/or charge variants of the formulation. In one embodiment, the stability of the liquid formulation disclosed herein can be tested in a high-temperature stress test, e.g., after storage at 40±2 °C for at least 1 week, 2 weeks or preferably 1 month, or after storage at 25±2 °C for at least 1 month or 2 months. In one embodiment, the shaking stability of the liquid formulation disclosed herein is tested by a shaking test.

In one embodiment, the stability of the liquid formulation disclosed herein is visually inspected after storage, wherein the liquid formulation disclosed herein remains a clear to slightly opalescent, colorless to pale yellow liquid free of particles in appearance. In one embodiment, no visible particles exist in the formulation upon visual inspection under a clarity detector. In one embodiment, the stability of the liquid formulation disclosed herein is tested after storage by determining the change in protein content, wherein the change rate in protein content is no more than 20%, preferably no more than 10%, e.g., 7-8%, preferably no more than 5% relative to an initial value on day 0 of storage, as measured, for example, by the ultraviolet spectrophotometry (UV) method. In one embodiment, the stability of the liquid formulation disclosed herein is tested after storage by determining the change in turbidity of the liquid formulation disclosed herein, wherein the change is no more than 0.04, preferably no more than 0.03, and more preferably no more than 0.02, relative to an initial value on day 0 of storage, as measured, for example, by the OD_{350 mm} method. In one embodiment, the stability of the liquid formulation disclosed herein is tested after storage by determining the change in purity of the liquid formulation disclosed herein, wherein the change in monomer purity is no more than 10%, e.g., no more than 5%, 4% or 3%, e.g., 1-2%, preferably no more than 1%, relative to an initial value on day 0 of storage, as measured by size exclusion-high performance liquid chromatography (SEC-HPLC). In one embodiment, the stability of the liquid formulation disclosed herein is tested after storage by determining the change in purity of the formulation disclosed herein, wherein the change in monomer purity is reduced by no more than 10%, e.g., no more than 5%, 4% or 3%, preferably no more than 2%, 1%, 0.5% or 0.1%, as measured by non-reduced and/or reduced capillary electrophoresis-sodium dodecyl sulfate (CE-SDS). In one embodiment, the stability of the liquid formulation disclosed herein is tested after storage by imaged capillary isoelectric focusing (iCIEF), wherein the change in charge variants (principal component, acidic component or basic component) of the antibody is no more than 2% relative to an initial value on day 0 of storage.

In one embodiment, the formulation is stable after storage, e.g., at 2-8 °C for at least 24 months, at room temperature for at least 3 months, or at 40±2 °C for 1 month, and preferably, has one or more of the following characteristics:
(i) a purity of the anti-LAG-3 antibody in the formulation greater than 90%, preferably greater than 95%, 96%, 97%, 98% or 99%, as measured by SEC-HPLC;
(ii) a purity greater than 90%, preferably greater than 95%, 96%, 97%, 98% or 99%, as measured by reduced or non-reduced CE-SDS;
(iii) changes ≤ 2% in components of the anti-LAG-3 antibody (i.e., principal component, acidic component and basic component) in the formulation relative to an initial value on day 0 of storage as measured by iCIEF; and
(iv) a relative binding activity of the anti-LAG-3 antibody in the formulation of 70-130%, e.g., 70%, 80%, 90%, 100%, 110%, 120% or 130%, relative to an initial value on day 0 of storage as measured by ELISA.

In one aspect, the present invention provides a delivery device comprising the liquid antibody formulation or the solid antibody formulation disclosed herein. In one embodiment, the delivery device disclosed herein is provided in the form of a pre-filled syringe comprising the liquid antibody formulation or the solid antibody formulation disclosed herein, e.g., for use in intravenous, subcutaneous, intradermal or intramuscular injection, or intravenous infusion.

In another aspect, the present invention provides a method for delivering the anti-LAG-3 antibody to a subject, e.g., a mammal, comprising administering the liquid antibody formulation or the solid antibody formulation disclosed herein to the subject, the delivery being implemented, e.g., using a delivery device in the form of a pre-filled syringe.

In another aspect, the present invention provides use of the liquid antibody formulation or the solid antibody formulation disclosed herein in preparing a delivery device (e.g., a pre-filled syringe) or a medicament for treating, preventing or delaying an LAG-3-expressing cancer (particularly a metastatic cancer), an immune-related disease and a T cell dysfunctional disease in a subject, particularly for treating, preventing or delaying an LAG-3-expressing cancer (particularly a metastatic cancer), e.g., various haematological tumors and solid tumors, such as colon cancer.

Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIG. 1 shows the effect of pH on purity of anti-LAG-3 antibody as measured by SEC-HPLC.
FIG. 2 shows the effect of pH on charge variants of anti-LAG-3 antibody as measured by iCIEF.
FIG. 3 shows the trend of change in charge variant-acidic component (by iCIEF) of anti-LAG-3 antibody formulas 1-7 in 2 weeks of high temperature stress test.
FIG. 4 shows the trend of change in charge variant-principal component (by iCIEF) of anti-LAG-3 antibody formulas 1-7 in 2 weeks of high temperature stress test.
FIG. 5 shows the trend of change in charge variant-basic component (by iCIEF) of anti-LAG-3 antibody formulas 1-7 in 2 weeks of high temperature stress test.
FIG. 6 shows the trend of change in turbidity (by OD_{350 nm} method) of anti-LAG-3 antibody formulas 8, 9 and 10 in high temperature stress test.
FIG. 7 shows the trend of change in purity (by SEC-HPLC) of anti-LAG-3 antibody formulas 8, 9 and 10 in high temperature stress test.
FIG. 8 shows the trend of change in purity (by reduced CE-SDS) of anti-LAG-3 antibody formulas 8, 9 and 10 in high temperature stress test.
FIG. 9 shows the trend of change in charge variant-acidic component (by iCIEF) of anti-LAG-3 antibody formulas 8, 9 and 10 in 1 month of high temperature stress test.
FIG. 10 shows the trend of change in charge variant-principal component (by iCIEF) of anti-LAG-3 antibody formulas 8, 9 and 10 in 1 month of high temperature stress test.
FIG. 11 shows the trend of change in charge variant-basic component (by iCIEF) of anti-LAG-3 antibody formulas 8, 9 and 10 in 1 month of high temperature stress test.
FIG. 12 shows the patterns of purity (by reduced CE-SDS) of anti-LAG-3 antibody formula 9 at the beginning and at month 1 in high temperature stress test.
FIG. 13 shows the mass spectrum (by LC-MS) of anti-LAG-3 antibody formula 9 at month 1 in high temperature stress test.
FIG. 14 shows the chromatograms of purity (by SEC-HPLC) of anti-LAG-3 antibody formula 10 at the beginning and at month 1 in high temperature stress test.

### DETAILED DESCRIPTION

Before the present invention is described in detail, it should be understood that the present invention is not limited to the particular methodology or experimental conditions described herein, as such methods and conditions may vary. Further, the terms used herein are for the purpose of describing particular embodiments only and are not intended to be limiting.

### Definitions

Unless defined otherwise, all technical and scientific terms used herein have the same meaning as commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "comprising" is intended to include the described elements, integers or steps, but not to exclude any other elements, integers or steps. As used herein, the term "comprise" or "comprising", unless indicated otherwise, also encompasses the situation where the entirety consists of the described elements, integers or steps. For example, when referring to an antibody variable region "comprising" a particular sequence, it is also intended to encompass an antibody variable region consisting of the particular sequence.

As used herein, the term "antibody" refers to a polypeptide comprising light chain and heavy chain variable regions of immunoglobulin that specifically identify and bind to an antigen. Preferably, the antibody disclosed herein is a full-length antibody, consisting of two heavy chains and two light chains, wherein each heavy chain consists of a heavy chain variable region (abbreviated herein as VH) and a heavy chain constant region, and each light chain consists of a light chain variable region (abbreviated herein as VL) and a light chain constant region. In some embodiments, the antibody disclosed herein can also refer to an antigen-binding fragment of an antibody.

The term "antibody formulation" refers to a preparation in a form that allows the biological activity of an antibody as an active ingredient to be exerted effectively, and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.

The term "anti-LAG-3 antibody formulation", herein also referred to as the "antibody formulation disclosed herein", refers to a preparation comprising an anti-LAG-3 antibody as an active ingredient and a pharmaceutically acceptable excipient. The anti-LAG-3 antibody, as the active ingredient, is suitable for therapeutic or prophylactic administration to a human or non-human animal after the anti-LAG-3 antibody is combined with the pharmaceutically acceptable excipient. The antibody formulation disclosed herein can be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the anti-LAG-3 antibody formulation is in the form of a liquid formulation.

A "stable" antibody formulation is a formulation where the antibody retains an acceptable degree of physical and/or chemical stability after storage under specific conditions. Although the antibody in the antibody formulation may not maintain 100% of its chemical structure after storage for a specific period of time, the antibody formulation is considered "stable" when the antibody typically maintains about 90%, about 95%, about 96%, about 97%, about 98%, or about 99% of its structure or function after storage for a specific period of time. In some specific embodiments, the antibody aggregation or degradation or chemical modification is barely detected in the anti-LAG-3 antibody formulation disclosed herein during manufacture, formulation, transportation and long-term storage, resulting in little or even no loss of biological activity of the anti-LAG-3 antibody and exhibiting high stability. In some embodiments, the anti-LAG-3 antibody formulation disclosed herein substantially retains its physical and chemical stability after storage. Preferably, the liquid formulation disclosed herein can remain stable at room temperature or at 40 °C for at least 1 month and/or at 2-8 °C for at least 24 months.

A variety of analytical techniques are known in the art for determining the stability of proteins, see, e.g., Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may encounter during manufacture, storage or transportation, and can also represent conditions that may accelerate the instability of the antibody in the antibody formulation during non-manufacture, storage or transportation. For example, the formulated anti-LAG-3 antibody formulation can be filled into a glass vial of a proper volume for shaking stress to test the shaking/shear stability of the antibody; alternatively, the formulated anti-LAG-3 antibody formulation can be filled into a glass vial to test the stability of the antibody under high temperature stress.

The antibody can be considered to "maintain its physical stability" in the formulation if the formulation does not exhibit aggregation, precipitation, turbidity and/or denaturation, or exhibits very little aggregation, precipitation, turbidity, and/or denaturation after storage for a period of time. Safety issues arise as the aggregation of antibodies in the formulation can potentially lead to an increased immune response in a patient. Accordingly, there is a need to minimize or prevent the aggregation of antibodies in the formulation. Light scattering methods can be used to determine visible aggregates in the formulation. SEC can be used to determine soluble aggregates in the formulation. In addition, the stability of the formulation can be indicated by visually inspecting the appearance, color and/or clarity of the formulation, or by detecting the turbidity of the formulation by the OD_{350 nm} method, or by determining the purity of the formulation by non-reduced CE-SDS. In one embodiment, the stability of the formulation is measured by determining the percentage of antibody monomer in the formulation after storage at a particular temperature for a particular period of time, wherein the higher the percentage of antibody monomer in the formulation, the higher the stability of the formulation.

An "acceptable degree" of physical stability can represent at least about 92% of anti-LAG-3 antibody monomer detected in the formulation after storage at a specific temperature for a specific period of time. In some embodiments, an acceptable degree of physical stability represents at least about 92%, 93%, 94%, 95%, 96%, 97%, 98% or 99% of anti-LAG-3 antibody monomer after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the physical stability is assessed, the specific temperature at which the pharmaceutical formulation is stored can be any temperature from about -80 °C to about 45 °C, e.g., at about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, a pharmaceutical formulation is considered stable if at least about 92%, 93%, 94%, 95%, 96%, 97%, 98% or 99% of the anti-LAG-3 antibody monomer is detected after storage at about 40±2 °C for 1 month or 4 weeks. A pharmaceutical formulation is considered stable if at least about 92%, 93%, 94%, 95%, 96%, 97%, 98% or 99% of the anti-LAG-3 antibody monomer is detected after storage at about 25 °C for 2 months. A pharmaceutical formulation is considered stable if at least about 92%, 93%, 94%, 95%, 96%, 97%, 98% or 99% of the anti-LAG-3 antibody monomer is detected after storage at about 5 °C for 9 months.

The antibody can be considered to "maintain its chemical stability" in the formulation if the antibody in the formulation does not exhibit significant chemical changes after storage for a period of time. Most of the chemical instability results from the formation of covalently modified forms of the antibody (e.g., charge variants of the antibody). Basic variants can be formed, for example, by aspartic acid isomerization, and N- and C-terminal modifications; acidic variants can be produced by deamidation, sialylation and glycation. Chemical stability can be assessed by detecting and/or quantifying chemically altered forms of the antibody. For example, charge variants of the antibody in the formulation can be detected by cation exchange chromatography (CEX) or imaged capillary isoelectric focusing (iCIEF). In one embodiment, the stability of the formulation is measured by determining the percentage change in charge variants of the antibody in the formulation after storage at a specific temperature for a specific period of time, wherein the smaller the change, the higher the stability of the formulation.

An "acceptable degree" of chemical stability can represent the percentage change in charge variants (e.g., principal component, acidic component or basic component) in the formulation of no more than 30%, e.g., 20%, after storage at a specific temperature for a specific period of time. In some embodiments, an acceptable degree of chemical stability can represent the percentage change in charge variant (acidic component) of no more than about 25%, 20%, 15%, 10%, 5%, 4%, 3%, 2% or 1% after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the chemical stability is assessed, the temperature at which the pharmaceutical formulation is stored can be any temperature from about -80 °C to about 45 °C, e.g., about -80 °C, about -30 °C, about -20 °C, about 0 °C, about 4-8 °C, about 5 °C, about 25 °C, or about 45 °C. For example, the pharmaceutical formulation can be considered stable if the percentage change in charge variant acidic component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 5 °C for 24 months. The pharmaceutical formulation can also be considered stable if the percentage change in charge variant acidic component is less than about 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 25 °C for 2 months. The pharmaceutical formulation can also be considered stable if the percentage change in charge variant acidic component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 40 °C for 1 month.

The term "lyophilized formulation" refers to a composition obtained or obtainable by a freeze-drying process of a liquid formulation. Preferably, it is a solid composition having a water content of less than 5%, preferably less than 3%.

The term "reconstituted formulation" refers to a liquid formulation obtained by dissolving and/or suspending a solid formulation (e.g., a lyophilized formulation) in a physiologically acceptable solution.

The term "room temperature" as used herein refers to a temperature from 15 °C to 30 °C, preferably from 20 °C to 27 °C, more preferably 25 °C.

"Stress conditions" refer to environments that are chemically and/or physically unfavorable to antibody proteins and may result in unacceptable destabilization of the antibody proteins. "High temperature stress" refers to storing the antibody formulation at room temperature or higher (e.g., 40±2 °C) for a period of time. The stability of the antibody formulation can be tested by the high-temperature stress accelerated test.

As used herein, the term "parenteral administration" refers to administrations other than enteral and topical administrations, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable anti-LAG-3 antibody formulation disclosed herein is administered parenterally to a subject. In one embodiment, the anti-LAG-3 antibody formulation disclosed herein is administered by subcutaneous, intradermal, intramuscular or intravenous injection to a subject.

### I. Antibody Formulation

The present invention provides a stable liquid antibody formulation comprising (i) a recombinant fully human anti-LAG-3 monoclonal antibody, (ii) a buffer, (iii) a stabilizer, and (iv) a surfactant, wherein the pH of the antibody formulation is about 5.5-7.5. In one preferred embodiment, the liquid antibody formulation disclosed herein is in the form of an injection.

### (i) Anti-LAG-3 antibody

An "anti-LAG-3 antibody" refers to an antibody that is capable of binding to an LAG-3 molecule with sufficient affinity such that the antibody can be used as a therapeutic and/or prophylactic agent targeting the LAG-3 molecule.

The antibody disclosed herein is a recombinant fully human antibody. The terms "fully human antibody" and "human antibody" are used interchangeably herein and refer to an antibody comprising variable regions in which both framework and CDR regions are derived from human germline immunoglobulin sequences, and if the antibody comprises constant regions, the constant regions are derived from human germline immunoglobulin sequences as well. The human antibody disclosed herein can include amino acids (e.g., mutations introduced by *in vitro* random or site-directed mutagenesis or *in vivo* somatic mutation) not encoded by human germline immunoglobulin sequences. However, as used herein, the term "human antibody" is not intended to include antibodies in which the CDR sequences are derived from the germline of other mammalian species (e.g., mice) and are grafted into human framework sequences. As used herein, the term "recombinant human antibody" includes all human antibodies that are prepared, expressed, produced or isolated by recombinant means. These recombinant human antibodies have variable regions in which framework regions and CDR regions are derived from human germline immunoglobulin sequences. However, in certain embodiments, the recombinant human antibodies can be subjected to *in vitro* mutagenesis (or *in vivo* somatic mutagenesis in the case of transgenic animals using human Ig sequences), and the amino acid sequences of the VH and VL regions of the resulting recombinant antibodies, although derived from and related to human germline VH and VL sequences, do not naturally occur in a human antibody germline repertoire. In some embodiments, the anti-LAG-3 antibody can specifically bind to human LAG-3 with a high affinity, e.g., with K_{D} of 10⁻⁷ M or less, preferably 10⁻⁹ M to 10⁻¹⁰ M as measured by biological optical interferometry, and thus mediates the efficient blocking of LAG-3 binding to its ligand.

In some embodiments, the anti-LAG-3 antibody disclosed herein comprises: a heavy chain variable region (VH) of SEQ ID NO: 7 or a VH having at least 90% identity thereto; and a light chain variable region (VL) of SEQ ID NO: 8 or a VL having at least 90% identity thereto. "Variable region" or "variable domain" is a domain in the heavy or light chain of an antibody that is involved in binding of the antibody to an antigen thereof. Generally, a heavy chain variable region (VH) and a light chain variable region (VL) can be further divided into hypervariable regions (HVRs, also known as complementarity determining regions (CDRs)) with more conservative regions (i.e., framework regions (FRs)) inserted therebetween. Each VH or VL consists of three CDRs and four FRs, arranged from amino-terminus to carboxyl-terminus in the following order: FR1, CDR1, FR2, CDR2, FR3, CDR3 and FR4.

In some embodiments, the anti-LAG-3 antibody disclosed herein comprises the VH CDR1, VH CDR2 and VH CDR3 sequences of the heavy chain variable region of SEQ ID NO: 7, and VL CDR1, VL CDR2 and VL CDR3 sequences of the light chain variable region of SEQ ID NO: 8. "Complementarity determining region", "CDR region" or "CDR" (used interchangeably herein with a "hypervariable region" (HVR)) is an amino acid region in the variable region of an antibody that is primarily responsible for binding to an epitope of an antigen. The CDRs of the heavy and light chains are generally referred to as CDR1, CDR2, and CDR3, and are numbered sequentially from the N-terminus. The CDRs located in the variable domain of an antibody heavy chain are referred to as VH CDR1, VH CDR2 and VH CDR3, while the CDRs located in the variable domain of an antibody light chain are referred to as VL CDR1, VL CDR2, and VL CDR3. Various schemes for determining the CDR sequence of a given VH or VL amino acid sequence are known in the art. For example, Kabat complementarity determining regions (CDRs) are determined based on sequence variability and are the most commonly used (Kabat et al., Sequences of Proteins of Immunological Interest, 5th Ed. Public Health Service, National Institutes of Health, Bethesda, Md. (1991)). Chothia scheme is based on the positions of structural loops (Chothia and Lesk, J. mol. biol. 196:901-917 (1987)). AbM HVRs are a compromise between Kabat HVRs and Chothia structural loops and are used by Oxford Molecular's AbM antibody modeling software. The "contact" HVRs are based on analysis of available complex crystal structures. HVRs can also be determined based on the same Kabat numbering position as the reference CDR sequences (e.g., exemplary CDRs disclosed herein). In one embodiment, the anti-LAG-3 antibody disclosed herein comprises a VH CDR1 of SEQ ID NO: 1, a VH CDR2 of SEQ ID NO. 2 and a VH CDR3 of SEQ ID NO: 3; and a VL CDR1 of SEQ ID NO: 4, a VL CDR2 of SEQ ID NO: 5 and a VL CDR3 of SEQ ID NO: 6.

In some embodiments, the anti-LAG-3 antibody disclosed herein can comprise a heavy chain variable region (VH) having at least 90%, 95%, 98%, or 99% or higher identity to SEQ ID NO: 7; and/or a light chain variable region (VL) having at least 90%, 95%, 98%, or 99% or higher identity to SEQ ID NO: 8. As used herein, the term "sequence identity" refers to the degree to which sequences are identical on a nucleotide-by-nucleotide or amino acid-by-amino acid basis in a comparison window. The "percent sequence identity" can be calculated by the following steps: comparing two optimally aligned sequences in a comparison window; determining a number of positions in which nucleic acid bases (e.g., A, T, C, G and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are the same in the two sequences to give the number of matched positions; dividing the number of matched positions by the total number of positions in the comparison window (i.e., the window size); and multiplying the result by 100 to give a percent sequence identity. Optimal alignment for determining the percent sequence identity can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithms necessary to achieve optimal alignment in a full-length sequence range or target sequence region being compared.

In some embodiments, the VH sequence of the antibody disclosed herein has no more than 10, preferably no more than 5, 4 or 3 different residues as compared to SEQ ID NO: 7, wherein preferably, the different residues are conservative amino acid substitutions. In some embodiments, the VL sequence of the antibody disclosed herein has no more than 10, preferably no more than 5, 4 or 3 different residues as compared to SEQ ID NO: 8, wherein preferably, the different residues are conservative amino acid substitutions. The "conservative substitution" refers to an amino acid alteration that results in the replacement of an amino acid with a chemically similar amino acid. Conservative substitution tables providing functionally similar amino acids are well known in the art. In any of the embodiments herein, in one preferred aspect, the conservatively substituted residue is from the conservative substitution Table A below, preferably the preferred substituted residues shown in Table A.

**Table A**

| Original residues | Exemplary substitution | Preferred conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In some embodiments, the antibody disclosed herein is an antibody in the form of IgG4. "Antibody in the form of IgG" refers to the heavy chain constant region of the antibody belonging to the IgG form. Heavy chain constant regions of all antibodies of the same type are identical, and heavy chain constant regions of antibodies of different types are different. For example, an antibody in the form of IgG4 refers to the Ig domain of its heavy chain constant region being an Ig domain of IgG4.

In one preferred embodiment, the anti-LAG-3 antibody disclosed herein is the anti-LAG-3 monoclonal antibody ADI-31853 disclosed in Chinese Patent Application No. CN201811561512.X (Dec. 19, 2018) comprising a heavy chain of SEQ ID NO: 9 and a light chain of SEQ ID NO: 10. In one embodiment, the anti-LAG-3 antibody is a purified IgG4 antibody produced by recombinant expression in 293 cells or CHO cells. Preferably, the antibody in the liquid formulation disclosed herein exhibits significant anti-tumor activity. For example, in mouse tumor models bearing mouse colon cancer cell CT26 (ATCC# CRL-2638) or human skin cancer cell A375 (ATCC# CRL-1619), administration of the antibody formulation disclosed herein may result in a tumor growth inhibition of about 50% or higher, e.g., 100%; and/or a tumor regression rate up to 60% or higher.

The amount of antibody or antigen-binding fragment thereof in the antibody formulation disclosed herein can vary with the specific desired characteristics of the formulation, the specific environment, and the specific purpose for which the formulation is used. In some embodiments, the antibody formulation is a liquid formulation, which may comprise about 1-100 mg/mL, preferably about 5-50 mg/mL, e.g., about 5, 10, 15, 20, 25, 30, 35, 40, 45 or 50 mg/mL, of anti-LAG-3 antibody.

### (ii) Buffer

Buffers are reagents that can control the pH of a solution within an acceptable range. In some embodiments, the buffer in the formulation disclosed herein can control the pH of the formulation disclosed herein at about 5.5-7.5, e.g., about 6.0-7.0, preferably 6.0±0.3. In one specific embodiment, the pH of the antibody formulation disclosed herein is about 5.5, 6.0, 6.5, 7.0 or 7.5, preferably about 6.0.

In some embodiments, the buffer in the formulation disclosed herein is selected from a citrate, a citrate solvate (e.g., citrate hydrate) and a combination thereof, e.g., sodium citrate, sodium citrate dihydrate and a combination thereof; preferably, the concentration of the buffer is about 1-10 mg/mL, preferably about 4-8 mg/mL, e.g., about 4, 4.5, 5, 5.5, 6, 6.5, 7, 7.5 or 8 mg/mL.

In one embodiment, the buffer in the formulation disclosed herein is sodium citrate or sodium citrate dihydrate at about 1-10 mg/mL, e.g., sodium citrate or sodium citrate dihydrate at about 2-8 mg/mL.

### (iii) Stabilizer

Suitable stabilizers for use in the present invention can be selected from saccharides, polyols and amino acids and combinations thereof. Saccharides used as stabilizers include, but are not limited to, sucrose and trehalose. Polyols used as stabilizers include, but are not limited to, sorbitol. Amino acids used as stabilizers include, but are not limited to, arginine and arginine hydrochloride. In some embodiments, the stabilizer is present in the liquid formulation disclosed herein in an amount of about 10-150 mg/mL, preferably about 15-100 mg/mL, e.g., about 15, 20, 30, 40, 50, 60, 70, 80, 90 or 100 mg/mL.

In one embodiment, the liquid formulation disclosed herein comprises sucrose as the stabilizer. The amount of sucrose in the liquid formulation disclosed herein can be about 20-100 mg/mL, preferably 40-90 mg/mL (e.g., 40, 50, 60, 70, 80 or 90 mg/mL).

In one embodiment, the liquid formulation disclosed herein comprises arginine and/or arginine hydrochloride as the stabilizer. The amount of arginine and/or arginine hydrochloride in the liquid formulations disclosed herein can be about 10-40 mg/mL, preferably about 15-25 mg/mL (e.g., about 15, 16, 17, 18, 19, 20, 21, 22, 23, 24 or 25 mg/mL).

In one embodiment, the liquid formulation disclosed herein comprises a combination of sucrose, arginine and/or arginine hydrochloride as the stabilizer. In such combination, sucrose can be present in an amount of about 20-100 mg/mL, preferably about 40-90 mg/mL (e.g., about 40, 50, 60, 70, 80, 90 mg/mL). In such combination, arginine and/or arginine hydrochloride can be present in an amount of about 10-40 mg/mL, preferably about 15-25 mg/mL (e.g., about 15, 16, 17, 18, 19, 20, 21, 22, 23, 24 or 25 mg/mL).

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation disclosed herein is a non-ionic surfactant, e.g., alkyl poly(ethylene oxide). Specific non-ionic surfactants that can be included in the formulation disclosed herein include, for example, polysorbates such as polysorbate-20, polysorbate-80, polysorbate-60 or polysorbate-40, Pluronic, and the like. In one preferred embodiment, the liquid formulation disclosed herein comprises polysorbate-80 as the surfactant.

The amount of surfactant in the antibody formulation disclosed herein can vary with the specific desired characteristics of the formulation, the specific environment, and the specific purpose for which the formulation is used. In some preferred embodiments, the formulation can comprise polysorbate surfactant (e.g., polysorbate-80) at about 0.1-1 mg/mL, preferably about 0.2-0.8 mg/mL, e.g., about 0.2, 0.3, 0.4, 0.5, 0.6, 0.7 or 0.8 mg/mL.

### (v) Other excipients

The liquid antibody formulation disclosed herein may or may not comprise other excipients.

In some embodiments, the liquid antibody formulation disclosed herein may or may not comprise an edetate (e.g., disodium edetate) and/or methionine.

In one embodiment, the liquid antibody formulation disclosed herein may or may not comprise an edetate (e.g., disodium edetate).

In one embodiment, the liquid antibody formulation disclosed herein may or may not comprise methionine.

In one embodiment, the liquid antibody formulation disclosed herein does not comprise an edetate (e.g., disodium edetate) and/or methionine, but has similar stability as compared to a corresponding formulation with an edetate (e.g., disodium edetate) and/or methionine added.

For other considerations, other excipients can also be used in the formulation disclosed herein. Such excipients include, for example, flavoring agents, antimicrobial agents, sweeteners, antistatic agents, antioxidants, gelatin, and the like. These and other known pharmaceutical excipients and/or additives suitable for use in the formulation disclosed herein are well known in the art, for example, as listed in *"*The Handbook of Pharmaceutical Excipients, 4th edition, edited by Rowe et al., American Pharmaceuticals Association (2003); and Remington: the Science and Practice of Pharmacy, 21st edition, edited by Gennaro, Lippincott Williams & Wilkins (2005)".

### II. Preparation of Formulation

The present invention provides a stable formulation comprising an antibody. The antibody used in the formulation disclosed herein can be prepared using techniques known in the art for the production of antibodies. For example, the antibody can be recombinantly prepared. In one preferred embodiment, the antibody disclosed herein is recombinantly prepared in 293 cells or CHO cells.

The use of antibodies as active ingredients in drugs is now very common. Techniques for purifying therapeutic antibodies to pharmaceutical grade are well known in the art. For example, Tugcu et al. (Maximizing productivity of chromatography steps for purification of monoclonal antibodies, Biotechnology and Bioengineering 99 (2008) 599-613) describes a monoclonal antibody three-column purification method in which ion exchange chromatography (anionic IEX and/or cationic CEX chromatography) is used after a protein A capture step. Kelley et al. (Weak partitioning chromatography for anion exchange purification of monoclonal antibodies, Biotechnology and Bioengineering 101 (2008) 553-566) describes a two-column purification method in which a weak partitioning anion exchange resin is used after protein A affinity chromatography.

Generally, monoclonal antibodies recombinantly produced can be purified using conventional purification methods to provide a drug with sufficient reproducibility and moderate purity for the formulation of antibody formulations. For example, after the antibody is secreted from the recombinant expression cells into the culture medium, the supernatant from the expression system can be concentrated using a commercially available protein concentration filter, e.g., Amicon ultrafiltration device. Then the antibody can be purified by methods such as chromatography, dialysis, and affinity purification. Protein A is suitable as an affinity ligand for the purification of IgG1, IgG2 and IgG4 antibodies. Other antibody purification methods, such as ion exchange chromatography, can also be used. After the antibody with sufficient purity is obtained, a formulation comprising the antibody can be prepared according to methods known in the art.

For example, the preparation can be performed by the following steps: (1) removing impurities such as cells from fermentation broth by centrifuging and clarifying after the fermentation to obtain a supernatant; (2) capturing an antibody using affinity chromatography (e.g., a protein A column with specific affinity for IgG1, IgG2 and IgG4 antibodies); (3) inactivating viruses; (4) purifying (usually CEX cation exchange chromatography can be adopted) to remove impurities in a protein; (5) filtering the viruses (to reduce the virus titer by, e.g., more than 4 log10); and (6) ultrafiltering/diafiltering (which can be used to allow the protein to be exchanged into a formulation buffer that is favorable for its stability and concentrated to a suitable concentration for injection). See, e.g., B. Minow, P. Rogge, K. Thompson, BioProcess International, Vol. 10, No. 6, 2012, pp. 48-57.

### III. Analytical Method of Formulation

During the storage of antibody formulations, antibodies may undergo aggregation, degradation or chemical modification, resulting in antibody heterogeneity (including size heterogeneity and charge heterogeneity), aggregates and fragments, etc., which may affect the quality of the antibody formulations. Accordingly, it is necessary to monitor the stability of antibody formulations.

Various methods are known in the art for testing the stability of antibody formulations. For example, the purity of the antibody formulation can be analyzed and the aggregation level of the antibody can be evaluated by methods such as non-reduced CE-SDS and SEC-HPLC; charge variants in the antibody formulation can be analyzed by capillary isoelectric focusing (cIEF), imaged capillary isoelectric focusing (iCIEF), ion exchange chromatography (IEX), and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by the ultraviolet spectrophotometry method (UV method).

Non-reduced CE-SDS is a method for determining the purity of monoclonal antibodies using a capillary as a separation channel. In CE-SDS, protein migration is driven by the surface charge caused by SDS binding, which is proportional to the molecular weight of the protein. Since all SDS-protein complexes have similar mass-to-charge ratios, electrophoretic separation based on the size or hydrodynamic radius of the molecules can be achieved in the molecular sieve gel matrix of the capillary. This method has been widely used to monitor the purity of denatured intact antibodies. Generally, in non-reduced CE-SDS, the sample is mixed with an SDS sample buffer and iodoacetamide. Then the mixture can be incubated at 68-72 °C for about 10-15 min and cooled to room temperature before the supernatant is centrifuged for analysis. The protein migration is detected using an ultraviolet detector to obtain an electrophoresis pattern. The purity of the antibody formulation can be calculated as the percentage of the IgG main peak area to the sum of all peak areas. For further description of CE-SDS, see, e.g., Richard R. et al., Application of CE SDS gel in development of biopharmaceutical antibody-based products, Electrophoresis, 2008, 29, 3612-3620.

Size exclusion-high performance liquid chromatography (SEC-HPLC) is another important method for the standardization and quality control of monoclonal antibodies. The method mainly separates molecules based on the differences in their size or hydrodynamic radius. Antibodies can be separated in three main forms by SEC-HPLC: high-molecular-weight species (HMMS), main peak (mainly antibody monomer), and low molecular-weight species (LMMS). The purity of antibody can be calculated as the percentage of the main peak area to the sum of all peak areas on the chromatogram. The percentage of antibody monomer in the formulation can be measured by SEC-HPLC, which gives information about the content of soluble aggregates and splices. For further description of SEC-HPLC, see, e.g., J. Pharm. Scien., 83:1645-1650, (1994); Pharm. Res., 11:485 (1994); J. Pharm. Bio. Anal., 15:1928 (1997); J. Pharm. Bio. Anal., 14:1133-1140 (1986). In addition, see also, e.g., R. Yang et al., High resolution separation of recombinant monoclonal antibodies by size exclusion ultra-high performance liquid chromatography (SE-UHPLC), Journal of Pharmaceutical and Biomedical Analysis (2015), http://dx.doi.org/10.1016/j.jpba.2015.02.032; and Alexandre Goyon et al., Protocols for the analytical characterization of therapeutic monoclonal antibodies, I-Non-denaturing chromatographic techniques, Journal of Chromatography, http://dx.doi.org/10.1016/j.jchromb.2017.05.010.

Imaged capillary isoelectric focusing (iCIEF) can be used to analyze the charge heterogeneity of monoclonal antibodies. This method can provide quantitative distribution of charge variants. iCIEF separates molecules based on the difference in their charge in a pH gradient (apparent pI value). In iCIEF, the separation column is typically a short capillary (e.g., silica capillary, 5 cm length, 100 µm I.D.), the proteins are focused in the capillary column at high voltage, and the focus is monitored online in real time by a whole column imaging detection system operating at 280 nM. One advantage of this technique is that various charge variants of an antibody sample can be simultaneously recorded by the whole column detection system. Generally, in iCIEF, the sample is mixed with urea and an iCIEF buffer containing methylcellulose, pI molecular weight standards, and ampholytes. Then after the sample has been focused for a period of time, the absorbance at 280 nm can be measured using an iCIEF column, such as a ProtionSimple assembled iCIEF column, on an iCIEF analyzer, such as an iCE280 analyzer (Protein Simple, Santa Clara, CA.), to obtain a spectrum of the focused mAb charge variants. In the iCEIF spectrum, protein-related peaks eluted before the main peak (i.e., principal component) are classified as acidic components, while protein-related peaks eluted after the main peak are classified as basic components. The relative amounts of the principal component, acidic component and basic component can be expressed as a percentage to the total peak area. For further description of iCIEF, see, e.g., Salas-Solano O et al., Robustness of iCIEF methodology for the analysis of monoclonal antibodies: an interlaboratory study, J Sep Sci. 2012 Nov.; 35(22):3124-9. doi: 10.1002/jssc.201200633. Epub 2012 Oct. 15; and Dada OO et al., Characterization of acidic and basic variants of IgG1 therapeutic monoclonal antibodies based on non-denaturing IEF fractionation, Electrophoresis. 2015 Nov.; 36(21-22):2695-2702. doi: 10.1002/elps.201500219. Epub 2015 Sep. 18.

The charge variants of the antibody in the antibody formulation can also be determined by cation exchange high performance liquid chromatography (CEX-HPLC). In this method, peaks eluted from the CEX-HPLC column earlier than the retention time of the main peak are labeled as "acidic peaks", while those eluted from the CEX-HPLC column later than the retention time of the main peak are labeled as "basic peaks".

Accelerated stability studies can be used to check the stability of products, which facilitates the screening of stable pharmaceutical formulations. For example, formulation samples can be placed at an elevated temperature, e.g., about 40±2 °C or 25±2 °C, for an accelerated stability study. The test indexes can include appearance, visible particles, protein content, turbidity, purity (SEC-HPLC and non-reduced CE-SDS) and charge variants (iCIEF).

A shaking test can be performed to investigate the shaking/shearing stability of the formulation. For example, formulation samples are aliquoted into vials, stoppered and capped, and then set out for the shaking test, e.g., shaking at 650 r/min for 3-5 days, after which the formulations are checked for appearance, protein content, turbidity, and purity.

In addition, the efficacy or biological activity of the antibody can be detected. For example, the ability of the antibody in the formulation to bind to its antigen can be tested. Various methods are known to those skilled in the art for quantifying the specific binding of an antibody to an antigen, such as immunoassay assays, e.g., ELISA.

The anti-LAG-3 antibody formulation disclosed herein is stable. In one embodiment, the purity of the anti-LAG-3 antibody in the antibody formulation disclosed herein is at least 90%, 91%, 92%, 93%, 94%, 95%, 96%, 97%, 98%, or 99% or higher after storage at about 25 °C, 37 °C, 40 °C or 45 °C for at least 1 month or 2 months, e.g., after storage at 40±2 °C for 1 month, as measured by the size exclusion chromatography or non-reduced CE-SDS. In one embodiment, at least 50%, preferably at least 55%, of the anti-ALG-3 antibody in the antibody formulation disclosed herein is in non-basic and non-acidic forms (i.e., the main peak or principal charge form) after storage at about 25 °C, 37 °C, 40 °C or 45 °C for at least 1 month or 2 months, e.g., after storage at 40±2 °C for 1 month, as measured by imaged capillary focusing.

### IV. Use of Formulation

The antibody formulation comprising an anti-LAG-3 antibody disclosed herein is useful for treating, ameliorating or preventing a variety of LAG-3-related diseases or disorders. As used herein, the "LAG-3-related disease or disorder" refers to a disease or disorder that can be treated (e.g., ameliorated) or prevented with the anti-LAG-3 antibody formulation disclosed herein. Any disease or disorder that can benefit from the treatment with the antibody formulation disclosed herein is suitable for the present invention.

In one aspect, the formulation comprising an anti-LAG-3 antibody disclosed herein can be used to modulate an immune response in a subject, particularly to restore, enhance, stimulate or increase an immune response in a subject. In some embodiments, the formulation comprising an anti-LAG-3 antibody disclosed herein can be used to restore, enhance or stimulate an antigen-specific T cell response in a subject, e.g., interleukin-2 (IL-2) or interferon-gamma (IFN-γ) production in an antigen-specific T cell response.

In another aspect, the formulation comprising an anti-LAG-3 antibody disclosed herein can be used to prevent or treat a tumor, such as a cancer including, but not limited to, a solid tumor, a hematological cancer (e.g., leukemia, lymphoma or myeloma), and metastatic lesions thereof, in a subject. In one embodiment, the cancer is a solid tumor. Examples of the solid tumor include a malignant tumor, e.g., sarcomas and carcinomas (e.g., adenocarcinomas) of various organ systems, such as those affecting the lung, the breast, the lymph, the gastrointestinal tract or colorectum, the genitalia, the genitourinary tract (e.g., kidney cells or bladder cells), the pharynx, CNS (e.g., brain cells, nerve cells, or glial cells), the skin (e.g., melanoma), the head and neck (e.g., head and neck squamous cell carcinoma (HNCC)), and the pancreas, including, for example, melanoma, colon cancer, gastric cancer, rectal cancer, renal cell carcinoma, breast cancer (e.g., breast cancer that does not express one, two, or all estrogen receptors, the progesterone receptor, or Her2/neu, such as triple negative breast cancer), liver cancer, lung cancer (e.g., non-small cell lung cancer (NSCLC), such as NSCLC with squamous and/or non-squamous histology, or small cell liver cancer), prostate cancer, head or neck cancer (e.g., HPV+ squamous cell carcinoma), small intestine cancer, and esophageal cancer. Examples of the hematological cancer include, but are not limited to, leukemia (e.g., myeloid leukemia, lymphoid leukemia, or chronic lymphocytic leukemia (CLL)), lymphoma (e.g., Hodgkin's lymphoma (HL), non-Hodgkin's lymphoma (NHL), diffuse large B-cell lymphoma (DLBCL), T-cell lymphoma, or mantle cell lymphoma (MCL)), and myeloma, e.g., multiple myeloma. The cancer may be at an early, intermediate or advanced stage, or may be a metastatic cancer. In one embodiment, the tumor is a gastrointestinal neoplasm, such as colon cancer.

In another aspect, the formulation comprising an anti-LAG-3 antibody disclosed herein can be used to prevent or treat an infectious disease in a subject. In some embodiments, the infectious disease is a chronic infection, e.g., caused by a pathogen selected from bacteria, viruses, fungi, and protozoa.

In another aspect, the formulation comprising an anti-LAG-3 antibody disclosed herein can be used to induce antibody-dependent cell-mediated cytotoxicity in a subject.

The present invention also provides use of the formulation disclosed herein in preparing a medicament for delivering the anti-LAG-3 antibody to a mammal, or for treating, preventing or ameliorating one or more of the diseases and disorders described above. Preferably, the mammal is a human.

The antibody formulation disclosed herein can be administered to a subject or a patient in a variety of pathways. For example, administration can be performed by infusion or by a syringe. Accordingly, in one aspect, the present invention provides a delivery device (e.g., a syringe) comprising the antibody formulation disclosed herein (e.g., a pre-filled syringe). The patient will receive an effective amount of the anti-LAG-3 antibody as the primary active ingredient, i.e., an amount sufficient to treat, ameliorate or prevent the disease or disorder of interest.

The therapeutic effect can include a reduction in physiological symptoms. The optimal effective amount and concentration of the antibody for any specific subject will depend on a variety of factors including the age, weight, health status and/or sex of the patient, the nature and extent of the disease, the activity of the specific antibody, its clearance by the body, as well as any other possible treatments administered in combination with the antibody formulation. For a specific case, the effective amount delivered can be determined based on the judgment of a clinician. Depending on the indication to be treated, an effective dose can range from about 0.005 mg/kg body weight to about 50 mg/kg body weight, or from about 0.1 mg/kg body weight to about 20 mg/kg body weight. In this aspect, the use of known antibody-based drugs can provide some guidance. The dosage can be a single-dose regimen or a multi-dose regimen.

The following examples are described to assist in understanding the present invention. The examples are not intended to be and should not be interpreted in any way as limiting the protection scope of the present invention.

### Abbreviations

CE-SDS: capillary electrophoresis-sodium dodecyl sulfate
ELISA: enzyme-linked immunosorbent assay
FLD: fluorescence detector
HPLC: high performance liquid chromatography
iCIEF: imaged capillary isoelectric focusing
SEC-HPLC: size exclusion-high performance liquid chromatography

### Examples

In order to develop a formula of recombinant fully human monoclonal anti-lymphocyte activation gene 3 (LAG-3) antibody injection for long-term stable storage and to ensure that the quality of a product is controllable within the shelf-life (at least 24 months), a formula screening test was designed and the effect of different excipients on the stability of LAG-3 antibody formulations was examined. The materials and methods used for the tests are as follows:

### Materials and methods

### 1.1. Materials

| Name | Grade | Manufacturer & site | Catalog No. | Criteria |
|---|---|---|---|---|
| Histidine | Pharmaceutical grade | Ajinomoto, Shanghai | NMPA Approval No. H20003296 | *Ch.P* (2015 Edition) |
| Sodium citrate (dihydrate) | Pharmaceutical grade | Merck, Germany | 1.37042.5000 | Ph Eur, BP, JP, USP |
| Sodium citrate (dihydrate) | Pharmaceutical grade | Xinning, Taishan | NMPA Approval No. H44024783 | *Ch.P* (2015 Edition) |
| Sodium citrate (monohydrate) | Pharmaceutical grade | Merck, Germany | 1.00242.5000 | Ph Eur, BP, JP, USP |
| Citric acid | Pharmaceutical grade | Merck, Germany | 1.00241.5000 | *Ch.P* (2015 Edition) |
| Sorbitol | Pharmaceutical grade | Roquette, French | H20110265 | EP, BP, NF, USP |
| Disodium edetate | Pharmaceutical grade | Avantor, USA | 8995-01 | EP, BP, JP, USP |
| Hydrochloric acid | Pharmaceutical grade | Merck, Germany | 1.00314.2508 | Ph Eur, BP, JP, NF |
| Sucrose | Pharmaceutical grade | Merck, Germany | 1.00892.9050 | *Ch.P* (2015 Edition), *USP* |
| Trehalose | Pharmaceutical grade | Pfanstiehl, USA | T-104-4 | USP/NF, EP, JP |
| Arginine | Pharmaceutical grade | Ajinomoto, Shanghai | NMPA Approval No. H20013215 | *Ch.P* (2015 Edition) |
| Arginine hydrochloride | Pharmaceutical grade | Ajinomoto, Shanghai | NMPA Approval No. H20003293 | *Ch.P* (2015 Edition) |
| Methionine | Pharmaceutical grade | Jianshun Biosciences, China | A021D | Ch.P |
| Polysorbate-80 | Pharmaceutical grade | Well, Nanjing | Jiangsu MPA Approval No. F15423203 | *Ch.P* (2015 Edition) |
| Capsule filter H4 | N/A | Sartorius, Germany | 5441307H4-OO-B | N/A |
| Platinum-cured silicone tubing | N/A | Nalgene, USA | 8600-0080 | N/A |
| 6R vial | N/A | Schott, Suzhou | 1142196 | N/A |
| 20 mm rubber stopper | N/A | West, Singapore | 7002-2354 | N/A |
| 20 mm aluminum-plastic cap | N/A | West, Singapore | 5420-1035 | N/A |

| | | | | |
|---|---|---|---|---|
| Note: N/A denotes "Not applicable". | | | | |

### 1.2. Instruments and equipment

| Name | Manufacturer & site | Model No. | Device No. |
|---|---|---|---|
| Electronic balance | Mettler, Switzerland | XPE30003S | PD-A1-247 |
| Electronic balance | Sartorius, Germany | BSA3202S | PD-A1-186 |
| pH meter | Mettler, Switzerland | FE20 | PD-A1-002 |
| pH meter | Mettler, Switzerland | FE20 | PD-A1-161 |
| Refrigerator (-20 °C) | Haier, Qingdao | BCD-290W | PD-A1-014 |
| Medical refrigerator | Haier, Qingdao | HYC-360 | PD-A1-166 |
| Medical refrigerator | Haier, Qingdao | HYC-360 | PD-A1-165 |
| Vortex mixer | VWR, USA | DVX-2500 | PD-A1-140 |
| Clarity detector | Tianda Tianfa, Tianjin | YB-2 | PD-A1-033 |
| Ultraviolet-visible spectrophotometer | Shimadzu, Japan | UV-1800 | AS-A1-037 |
| Benchtop refrigerated centrifuge | Thermo, USA | SL16R | PD-A1-082 |

### 1.3. Test items and methods for formulation stability

The antibody formulation was tested for the following items: (1) the appearance and the presence of visible particles; (2) the protein content in the formulation determined by the ultraviolet method (UV method); (3) the turbidity of the formulation determined by the OD_{350 nm} method; (4) the purity of the antibody formulation determined by size exclusion-high performance liquid chromatography (SEC-HPLC) and expressed as the percentage of the main peak area to the sum of all peak areas; (5) the purity of the antibody formulation determined by reduced capillary electrophoresis-sodium dodecyl sulfate (reduced CE-SDS) and/or non-reduced capillary electrophoresis-sodium dodecyl sulfate (non-reduced CE-SDS) and expressed as the percentage of the monomer peak area to the sum of all peak areas; (6) the charge variants in the antibody formulation determined by imaged capillary isoelectric focusing (iCIEF) and expressed as the percentage of the principal component, acidic component and basic component; and (7) the relative binding activity of the anti-LAG-3 antibody in the antibody formulation determined by immunoassay, e.g., direct ELISA.

### Detection of visible particles

The visible particles in the sample were detected using a clarity detector (model No. YB-2, Tianda Tianfa, Tianjin) according to the method described in the National Pharmacopoeia Committee, the Pharmacopoeia of the People's Republic of China (2015 edition, volume IV General Rules 0904 "Test for Visible Particles"), Beijing, China Medical Science Press, 2015.

### Determination of protein content

The protein content in the sample was determined using an ultraviolet spectrophotometer (model No. UV-1800, Shimadzu, Japan).

### Determination of turbidity

The turbidity in the sample was determined by measuring the absorbance at 350 nm using an ultraviolet spectrophotometer (model No. UV-1800, Shimadzu, Japan).

### Purity (SEC-HPLC)

Separation was performed using an SEC column with a phosphate buffer (3.12 g of sodium dihydrogen phosphate dihydrate, 8.77 g of sodium chloride and 34.84 g of arginine were dissolved in ultra-pure water, the pH was adjusted to 6.8 by adding hydrochloric acid, and the volume was brought to 1000 mL) as the mobile phase. The chromatographic column protective solution was 0.05% (w/v) NaN₃, the sample injection volume was 50 µL, the flow rate was 0.5 mL/min, the collection time was 30 min, the column temperature was 25 °C, and the detection wavelength was 280 nm. A sample was diluted to 2 mg/mL by ultra-pure water for use as a sample solution. A formulation buffer was diluted in the same manner as described above to prepare a blank solution. The blank solution and the sample solution were separately loaded on a liquid chromatographic column in an amount of 50 µL for the determination.

### Purity (reduced CE-SDS)

The determination was conducted by capillary gel electrophoresis. The capillary was an uncoated capillary having an inner diameter of 50 µm, a total length of 30.2 cm and an effective length of 20.2 cm. Before electrophoresis, the capillary column was washed with 0.1 mol/L sodium hydroxide, 0.1 mol/L hydrochloric acid, ultra-pure water, and electrophoresis gel at 70 psi. The sample was diluted to 2.0 mg/mL with an appropriate amount of ultra-pure water. 50 µL of the diluted sample was transferred into a 1.5-mL centrifuge tube, and 45 µL of sample buffer at pH 6.5 (0.32 g of citric acid monohydrate and 2.45 g of disodium phosphate dodecahydrate were dissolved in 45 mL of ultra-pure water, and the volume was brought to 50 mL to prepare the citrate-phosphate buffer; 80 µL of 10% (w/v) sodium dodecyl sulfate solution was added to 200 µL of the buffer, the volume was brought to 1 mL with water, and the mixture was well mixed to obtain the sample buffer), 1 µL of internal standard (10 kDa protein, 5 mg/mL; Beckman Coulter, Catalog No. 390953) and 5 µL of β-mercaptoethanol were added. The mixture was well mixed, heated at 70±2 °C for 10±2 min, cooled to room temperature, and transferred to a sample bottle for future use as a sample solution. A formulation buffer of the same volume as the sample was processed by the same method as above to prepare the blank solution. Conditions for sample injection: -5 kV for 20 s; separation voltage: -15 kV for 35 min. The capillary column temperature was controlled at 25 °C and the detection wavelength was 220 nm.

### Purity (non-reduced CE-SDS)

The determination was conducted by capillary gel electrophoresis. The capillary was an uncoated capillary having an inner diameter of 50 µm, a total length of 30.2 cm and an effective length of 20.2 cm. Before electrophoresis, the capillary column was washed with 0.1 mol/L sodium hydroxide, 0.1 mol/L hydrochloric acid, ultra-pure water, and electrophoresis gel at 70 psi. A sample was diluted to 2.0 mg/mL with an appropriate amount of ultra-pure water. 50 µL of the diluted sample was transferred into a 1.5-mL centrifuge tube, and 45 µL of sample buffer at pH 6.5 (0.32 g of citric acid monohydrate and 2.45 g of disodium phosphate dodecahydrate were dissolved in 45 mL of ultra-pure water, and the volume was brought to 50 mL to prepare the citrate-phosphate buffer; 80 µL of 10% (w/v) sodium dodecyl sulfate solution was added to 200 µL of the buffer, the volume was brought to 1 mL with water, and the mixture was well mixed to obtain the sample buffer), 1 µL of internal standard (10 kDa protein, 5 mg/mL; Beckman Coulter, Catalog No. 390953) and 5 µL of 250 mmol/L NEM solution (62 mg of *N*-ethylmaleimide was dissolved in 2 mL of ultra-pure water) were added. The mixture was well mixed, heated at 70±2 °C for 10±2 min, cooled to room temperature, and transferred to a sample bottle for future use as a sample solution. A formulation buffer of the same volume as the sample was processed by the same method as above to prepare the blank solution. Conditions for sample injection: -5 kV for 20 s; separation voltage: -15 kV for 35 min. The capillary column temperature was controlled at 25 °C and the detection wavelength was 220 nm.

### Charge variants (iCIEF)

The determination was conducted by imaged capillary isoelectric focusing (icIEF). The inner diameter of the capillary was 100 µm, and the total length was 5 cm. The capillary column was rinsed with 0.5% methyl cellulose solution (hereinafter abbreviated as MC solution) and ultra-pure water before electrophoresis. The sample was injected in vacuum for 55 s, the pre-focusing was conducted at 1.5 kV for 1 min, the focusing was conducted at 3 kV for 8 min, the sample injection time was 55 s, the temperature of the sample tray was 10 °C, the capillary column temperature was room temperature, and the detection wavelength was 280 nm. The cathodic stabilizer was 500 mmol/L arginine, and the anodic stabilizer was 200 mmol/L iminodiacetic acid. 3 mol/L urea was added to improve the protein solubility, and 0.5% MC solution was added to decrease the adhesion between the protein and the capillary. The sample was diluted to 0.5 mg/mL with water, 20 µL of the diluted test sample was added to 83 µL of a premixed solution, and the mixture was well mixed to prepare a sample solution. The same procedures were performed using the formulation buffer to prepare a blank solution.

### Relative binding activity (direct ELISA)

Streptavidin (Thermo, Catalog No.: 21125) was diluted to 1 µg/mL with 1× PBS, and immobilized on a 96-well microplate in an amount of 100 µL/well at 37 °C for 2 h. After the plate was washed, a blocking solution (5% FBS, 300 µL/well) was added for 2 h of blocking at 37 °C. Biotinylated LAG-3 (Sino biological, Catalog No.: 16498-HNAH-B) was diluted to 0.5 µg/mL with 1× PBS, and immobilized on a 96-well microplate in an amount of 100 µL/well at 37 °C for 0.5 h. The anti-LAG-3 antibody was diluted to 40 µg/mL with 2% FBS, transferred in a volume of 100 µL/well, and serially diluted by 4 folds to the 12^{th} concentration (0.01-10000 ng/mL). The serially diluted samples were added to a washed plate at 100 µL/well, and incubated at 37 °C for 30 min in a thermostatic incubator. After the plate was washed, HRP-conjugated goat anti-human IgG-Fc fragment (BETHYL, USA, Catalog No. A80-104P) diluted with 2% FBS was added as the secondary antibody (30000-fold dilution, 100 µL/well), and the plate was incubated for 20 min at 37 °C. After the plate was washed, 100 µL of TMB was added, and after 10 min of chromogenic reaction, 100 µL of 1 mol/L H₂SO₄ was added to each well to terminate the reaction. The OD value at 450 nm was measured with 620 nm being the reference wavelength. A curve of concentration vs. OD_{450 nm}-OD_{620 nm} of the serially diluted samples was plotted, and the EC₅₀ representing the binding activity of anti-LAG-3 antibody to LAG-3 was calculated by Prism four-parameter fitting.

### Example 1. Preparation and Purification of Anti-LAG-3 Antibody

A novel anti-LAG-3 antibody ADI-31853 specifically binding to LAG-3 was prepared and purified as described in CN201811561512.X, which consists of a heavy chain sequence of SEQ ID NO: 9 and a light chain sequence of SEQ ID NO: 10 and is an IgG4 antibody. Briefly, the anti-LAG-3 antibody ADI-31853 was recombinantly expressed in 293 cells or CHO cells and purified. The samples used in the formula screening test was purified by CEX (cation exchange chromatography) to give samples with protein contents of about 11.5 mg/mL to about 25.0 mg/mL.

### Example 2: Test for Effect of pH on Stability of Formulation

This example investigated the stability of formulations comprising anti-LAG-3 antibody at pH 5.0-8.0.

### 2.1. Procedures:

A total of 7 pH values were designed, as detailed in Table 1. Buffers of the formulas were prepared, and the solvent of anti-LAG-3 antibody was replaced by the buffers via ultrafiltration. After buffer exchange, the protein content of the formulations was adjusted to about 20 mg/mL and polysorbate-80 was added. The solutions were filtered and filled into vials. The vials were stopped and capped, and were subjected to high-temperature stress stability test, where the purity of the anti-LAG-3 antibody was measured by SEC-HPLC and the charge variants were measured by iCIEF.

**Table 1. Formulas**

| No. | Formulas |
|---|---|
| Formula A | 20 mM histidine, 5% (w/v) sorbitol, 0.20 mg/mL polysorbate-80, pH 5.0 |
| Formula B | 20 mM histidine, 5% (w/v) sorbitol, 0.20 mg/mL polysorbate-80, pH 5.5 |
| Formula C | 20 mM histidine, 5% (w/v) sorbitol, 0.20 mg/mL polysorbate-80, pH 6.0 |
| Formula D | 20 mM histidine, 5% (w/v) sorbitol, 0.20 mg/mL polysorbate-80, pH 6.5 |
| Formula E | 20 mM histidine, 5% (w/v) sorbitol, 0.20 mg/mL polysorbate-80, pH 7.0 |
| Formula F | 20 mM histidine, 5% (w/v) sorbitol, 0.20 mg/mL polysorbate-80, pH 7.5 |
| Formula G | 20 mM histidine, 5% (w/v) sorbitol, 0.20 mg/mL polysorbate-80, pH 8.0 |

The detailed test conditions and sampling schedule are shown in Table 2.

**Table 2. Test conditions and sampling schedule**

| Test name | Formulas | Test scheme and sampling time points |
|---|---|---|
| High temperature stress test | Formulas A-G | The solutions were let standing at 40±2 °C, and samples were collected at day 0, week 1, week 2 and month 1. |

### 2.2. Criteria

According to the knowledge of the product and the precision of the instrument and the method, criteria for determining the absence of changes in sample test indexes as compared to initial values were set, so as to determine whether the sample changed, as detailed in Table 3.

**Table 3. Criteria for absence of quality change**

| Test items | Criteria |
|---|---|
| Appearance (visual inspection) | Clear to slightly opalescent, colorless to pale yellow liquid free of particles |
| Visible particles (Test for visible particles) | Conforms to the General Rule 0904 of the Pharmacopoeia of the People's Republic of China (2015 edition, volume IV) |
| Protein content (UV method) | Change rate ≤ 10% |
| Purity (SEC-HPLC) | Change in main peak purity ≤ 1% |
| Purity (reduced CE-SDS) | Change in main peak purity ≤ 2% |
| Purity (non-reduced CE-SDS) | Change in main peak purity ≤ 2% |
| Charge variants (iCIEF) | Changes in principal component, acidic component and basic component ≤ 2% |
| Charge variants (CEX-HPLC) | Changes in principal component, acidic component and basic component ≤ 2% |
| Relative binding activity (direct ELISA) | Should be 70-130% |

### 2.3. Results

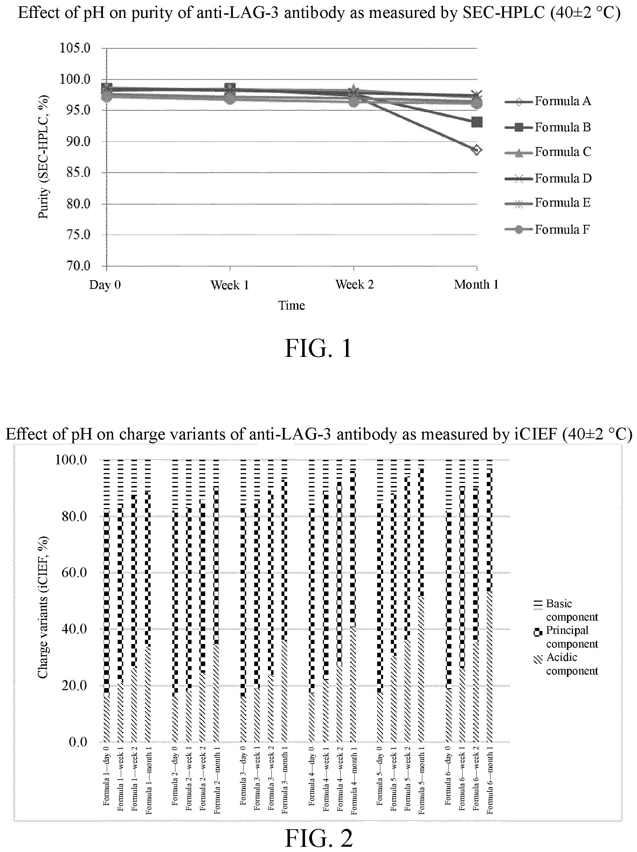

The results of test for effect of pH on stability of formulation are shown in Table 4. The trend of change in purity of the anti-LAG-3 antibody measured by SEC-HPLC is shown
in FIG. 1, and the trend of change in charge variants of the anti-LAG-3 antibody measured by iCIEF is shown in FIG. 2. As can be seen from Table 4, FIG. 1 and FIG. 2,
after the formulations were let standing at 40±2 °C for one month, the anti-LAG-3 antibody was stable in solutions at pH 5.5, pH 6.0, pH 6.5, pH 7.0 and pH 7.5. That is,
the formulation could be performed at a pH in the range of 5.5-7.5. Therefore, in subsequent studies, LAG 3 antibody formulations were formulated at pH 6.0.Table 4.

### Results of test for effect of pH on stability of formulation (40±2 °C)

| Sample name | Purity (SEC-HPLC, %) | | | | Charge variants (iCIEF^{∗}, %) | | | | | | | | | | | |
|---|---|---|---|---|---|---|---|---|---|---|---|---|---|---|---|---|
| | | | | | Acidic component | | | | Principal component | | | | Basic component | | | |
| | Day 0 | Week 1 | Week 2 | Month 1 | Day 0 | Week 1 | Week 2 | Month 1 | Day 0 | Week 1 | Week 2 | Month 1 | Day 0 | Week 1 | Week 2 | Month 1 |
| Formula A | 98.3 | 98.5 | 97.4 | 88.6 | 16.6 | 21.1 | 26.2 | 33.9 | 65.5 | 63.0 | 60.9 | 54.8 | 18.0 | 15.9 | 12.9 | 11.4 |
| Formula B | 98.5 | 98.5 | 97.6 | 93.1 | 16.3 | 18.3 | 24.6 | 34.4 | 65.8 | 65.0 | 61.3 | 55.8 | 17.9 | 16.6 | 14.1 | 9.8 |
| Formula C | 98.7 | 98.4 | 98.3 | 97.1 | 16.3 | 19.2 | 23.7 | 36.0 | 66.7 | 66.3 | 65.4 | 57.0 | 17.0 | 14.6 | 11.0 | 7.1 |
| Formula D | 98.5 | 98.2 | 97.8 | 97.4 | 17.4 | 22.0 | 27.1 | 40.7 | 65.3 | 66.7 | 65.2 | 55.4 | 17.3 | 11.4 | 7.7 | 3.9 |
| Formula E | 97.6 | 97.2 | 97.0 | 96.5 | 17.5 | 30.6 | 36.3 | 51.5 | 66.9 | 57.5 | 57.9 | 45.6 | 15.6 | 11.8 | 5.8 | 2.9 |
| Formula F | 97.2 | 96.8 | 96.4 | 96.1 | 18.9 | 26.2 | 35.4 | 53.2 | 63.0 | 64.1 | 54.5 | 43.5 | 18.0 | 9.7 | 10.1 | 3.4 |
| Formula G | 97.1 | 94.1 | 89.3 | 79.3 | 20.3 | 88.4 | 100.0 | N/A | 69.2 | 11.6 | 0.0 | N/A | 10.5 | 0.0 | 0.0 | N/A |

| | | | | | | | | | | | | | | | | |
|---|---|---|---|---|---|---|---|---|---|---|---|---|---|---|---|---|
| Note: N/A denotes no investigation designed. | | | | | | | | | | | | | | | | |

### Example 3. Formula Screening Test (I)

### 3.1. Procedures

A total of 7 formulas were designed, as detailed in Table 5. Buffers of the formulas were prepared as in Table 5. The solvent of antibody was replaced by solutions of the formulations via ultrafiltration. After buffer exchange, proteins of the formulas were diluted to about 20 mg/mL and polysorbate-80 was added. The solutions were filtered and filled into vials. The vials were stopped and capped, and were subjected to stability test. Stability tests used methods including high temperature stress test, shaking test, freezing-thawing test and illumination test. The indexes included appearance, visible particles, protein content (UV method), turbidity (OD_{350 nm} method), purity (SEC-HPLC and non-reduced CE-SDS), charge variants (iCIEF) and relative binding activity (direct ELISA).

**Table 5. Formulas for screening test (I)**

| No. | Formulas |
|---|---|
| Formula 1 | 5.88 mg/mL sodium citrate (dihydrate), 50.00 mg/mL sorbitol, 1.49 mg/mL methionine, 0.30 mg/mL polysorbate-80, pH 6.0 |
| Formula 2 | 5.88 mg/mL sodium citrate (dihydrate), 50.00 mg/mL sorbitol, 0.0075 mg/mL disodium edetate, 0.30 mg/mL polysorbate-80, pH 6.0 |
| Formula 3 | 1.55 mg/mL histidine, 50.00 mg/mL sorbitol, 0.30 mg/mL polysorbate-80, pH 6.0 |
| Formula 4 | 5.88 mg/mL sodium citrate (dihydrate), 50.00 mg/mL sorbitol, 0.30 mg/mL polysorbate-80, pH 6.0 |
| Formula 5 | 5.88 mg/mL sodium citrate (dihydrate), 80.00 mg/mL sucrose, 0.30 mg/mL polysorbate-80, pH 6.0 |
| Formula 6 | 5.88 mg/mL sodium citrate (dihydrate), 80.00 mg/mL trehalose, 0.30 mg/mL polysorbate-80, pH 6.0 |
| Formula 7 | 5.88 mg/mL sodium citrate (dihydrate), 17.42 mg/mL arginine, 0.30 mg/mL polysorbate-80, pH 6.0 |

The detailed test conditions and sampling schedule are shown in Table 6.

**Table 6. Test conditions and sampling schedule**

| Test name | Formulas | Test scheme and sampling time points |
|---|---|---|
| High temperature stress test | Formulas 1-7 | The solutions were let standing at 40±2 °C, and samples were collected at day 0 and week 2. |
| Shaking test | Formulas 1, 2 and 4 | 650 rpm, 25±2 °C, away from light; samples were collected on days 0, 1, 3 and 5 |
| Freezing-thawing test | | A cycle consisted of a freezing at -20±2 °C for 1 day and a thawing at 5±3 °C; samples were collected after cycles 0, 3 and 6. |
| Illumination test | | 25±2 °C, 600±50 Lux irradiation; samples were collected on days 0, 3 and 5 |

### 3.2. Criteria

See Section 2.2 and Table 3 above.

### 3.3. Results

### (1) High temperature stress test

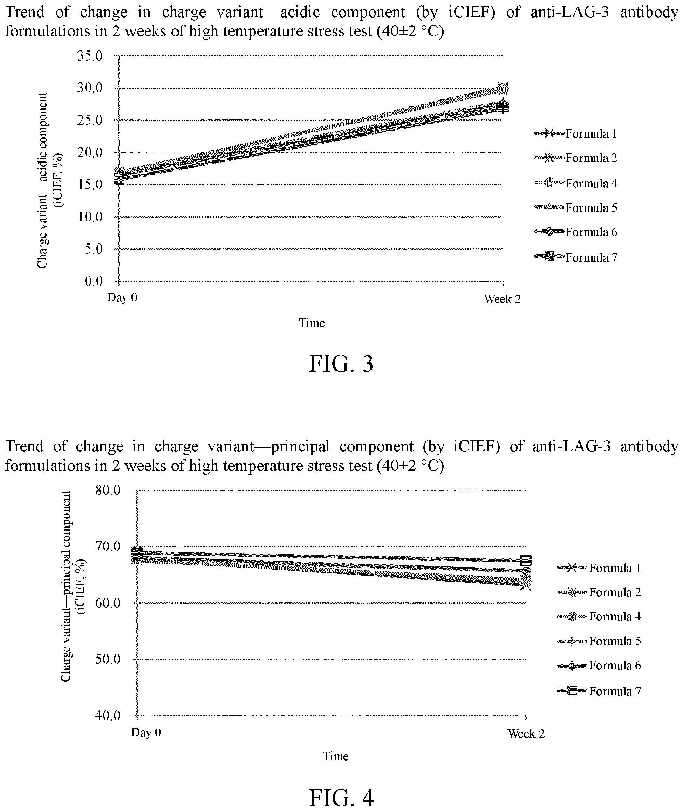

The results of high temperature stress formula screening are detailed in Table 7 and FIGs. 3-5.

After standing at 40±2 °C for 2 weeks, white precipitate was observed in formula 3 only. The formulas 1, 2, 4, 5, 6 and 7 were all qualified in aspects of appearance and visible particles; protein content (UV method) and purity (SEC-HPLC and non-reduced CE-SDS) remained unchanged; for charge variants, acid component (iCIEF) increased, and basic component (iCIEF) decreased, wherein acidic components of formulas 5, 6 and 7 showed smaller changes relative to formulas 1, 2 and 4; principal components (iCIEF) of formulas 5 and 7 remained unchanged according to the criteria in Table 3 above.

**Table 7. Results of high temperature stress test for formula screening (40±2 °C)**

| Sample name | Protein content (UV method, mg/mL) | | Purity (%) | | | | Charge variants (iCIEF, %) | | | | | |
|---|---|---|---|---|---|---|---|---|---|---|---|---|
| | | | SEC-HPLC | | Non-reduced CE-SDS | | Acidic component | | Principal component | | Basic component | |
| | Day 0 | Week 2 | Day 0 | Week 2 | Day 0 | Week 2 | Day 0 | Week 2 | Day 0 | Week 2 | Day 0 | Week 2 |
| Formula 1 | 20.8 | 20.8 | 98.3 | 97.8 | 98.7 | 98.3 | 16.6 | 30.1 | 67.9 | 63.2 | 15.5 | 6.7 |
| Formula 2 | 20.4 | 20.8 | 98.2 | 97.5 | 98.9 | 98.7 | 16.9 | 29.7 | 67.5 | 64.1 | 15.6 | 6.2 |
| Formula 3 | 20.1 | N/A | 98.3 | N/A | 99.0 | N/A | 15.5 | N/A | 68.8 | N/A | 15.6 | N/A |
| Formula 4 | 20.7 | 20.6 | 98.2 | 97.5 | 98.9 | 98.7 | 16.9 | 29.9 | 67.9 | 63.8 | 15.3 | 6.3 |
| Formula 5 | 20.8 | 20.6 | 98.2 | 97.7 | 98.9 | 98.7 | 16.8 | 27.8 | 67.6 | 65.7 | 15.6 | 6.5 |
| Formula 6 | 19.6 | 19.9 | 98.2 | 97.7 | 99.0 | 98.4 | 16.5 | 27.4 | 68.0 | 65.7 | 15.6 | 7.0 |
| Formula 7 | 20.3 | 20.4 | 98.3 | 98.2 | 99.0 | 98.4 | 15.8 | 26.8 | 68.9 | 67.5 | 15.2 | 5.7 |

| | | | | | | | | | | | | |
|---|---|---|---|---|---|---|---|---|---|---|---|---|
| Note: N/A denotes no investigation designed. | | | | | | | | | | | | |

### (2) Shaking test

The results for shaking test for formula screening are detailed in Table 8. The results showed that after shaking at 650 rpm for 5 days, formulas 1, 2 and 4 were qualified in aspects of appearance and visible particles; protein content (UV method), turbidity (OD_{350 nm} method), purity (SEC-HPLC and non-reduced CE-SDS), charge variants (iCIEF) and relative binding activity (direct ELISA) remained unchanged.

**Table 8. Results of shaking test for formula screening**

| Test items | | Time (day) | Sample name | | |
|---|---|---|---|---|---|
| | | | Formula 1 | Formula 2 | Formula 4 |
| Protein content (UV method, mg/mL) | | Day 0 | 20.2 | 19.7 | 20.9 |
| | | Day 1 | 20.4 | 19.8 | 21.1 |
| | | Day 3 | 20.1 | 19.4 | 20.4 |
| | | Day 5 | 20.4 | 19.6 | 20.9 |
| Turbidity (OD_{350 nm} method) | | Day 0 | 0.076 | 0.074 | 0.080 |
| | | Day 1 | 0.077 | 0.076 | 0.080 |
| | | Day 3 | 0.079 | 0.074 | 0.083 |
| | | Day 5 | 0.082 | 0.079 | 0.082 |
| Purity (%) | SEC-HPLC | Day0 | 98.1 | 98.1 | 98.1 |
| | | Day1 | 98.1 | 98.1 | 98.0 |
| | | Day3 | 98.1 | 97.9 | 97.9 |
| | | Day5 | 98.0 | 97.9 | 97.8 |
| | Non-reduced CE-SDS | Day0 | 98.7 | 98.8 | 98.9 |
| | | Day1 | 98.8 | 99.1 | 99.0 |
| | | Day3 | 98.6 | 98.5 | 98.9 |
| | | Day5 | 98.7 | 99.0 | 98.9 |
| Charge variants (iCIEF, %) | Acidic component | Day0 | 15.7 | 16.1 | 16.7 |
| | | Day1 | 16.4 | 16.6 | 16.9 |
| | | Day3 | 17.2 | 16.9 | 17.3 |
| | | Day5 | 17.5 | 17.6 | 17.6 |
| | Principal component | Day0 | 69.0 | 68.3 | 68.0 |
| | | Day1 | 68.2 | 68.0 | 67.9 |
| | | Day3 | 69.1 | 68.3 | 68.3 |
| | | Day5 | 68.5 | 68.4 | 68.4 |
| | Basic component | Day0 | 15.3 | 15.6 | 15.3 |
| | | Day1 | 15.4 | 15.4 | 15.3 |
| | | Day3 | 13.8 | 14.9 | 14.4 |
| | | Day5 | 14.0 | 14.0 | 14.1 |
| Relative binding activity (direct ELISA, %) | | Day 0 | 123 | 96 | 85 |
| | | Day 1 | N/A | N/A | N/A |
| | | Day 3 | N/A | N/A | N/A |
| | | Day 5 | 104 | 102 | 94 |

| | | | | | |
|---|---|---|---|---|---|
| Note: N/A denotes no investigation designed. | | | | | |

### (3) Freezing-thawing test

The results of freezing-thawing test for formula screening are detailed in Table 9. After 6 freezing/thawing cycles, formulas 1, 2 and 4 were qualified in appearance and visible particles; protein content (UV method), turbidity (OD_{350 nm} method), purity (SEC-HPLC and non-reduced CE-SDS), charge variants (iCIEF) and relative binding activity (direct ELISA) remained unchanged.

**Table 9. Results of freezing-thawing test for formula screening**

| Test items | | Number of freezing/thaw ing cycles | Sample name | | |
|---|---|---|---|---|---|
| | | | Formula 1 | Formula 2 | Formula 4 |
| Protein content (UV method, mg/mL) | | 0 | 20.2 | 19.7 | 20.9 |
| | | 3 | 20.6 | 20.0 | 21.1 |
| | | 6 | 20.5 | 19.8 | 21.0 |
| Turbidity (OD_{350 nm} method) | | 0 | 0.076 | 0.074 | 0.080 |
| | | 3 | 0.077 | 0.076 | 0.082 |
| | | 6 | 0.080 | 0.077 | 0.079 |
| Purity (%) | SEC-HPLC | 0 | 98.1 | 98.1 | 98.1 |
| | | 3 | 98.1 | 98.1 | 98.1 |
| | | 6 | 98.1 | 98.1 | 98.0 |
| | Non-reduced CE-SDS | 0 | 98.7 | 98.8 | 98.9 |
| | | 3 | 98.7 | 99.1 | 99.0 |
| | | 6 | 99.0 | 98.9 | 98.9 |
| Charge | Acidic component | 0 | 15.7 | 16.1 | 16.7 |
| variants (iCIEF, %) | | 3 | 16.7 | 16.7 | 16.1 |
| | | 6 | 16.9 | 16.9 | 17.0 |
| | Principal component | 0 | 69.0 | 68.3 | 68.0 |
| | | 3 | 67.9 | 68.3 | 68.5 |
| | | 6 | 68.0 | 67.9 | 68.3 |
| | Basic component | 0 | 15.3 | 15.6 | 15.3 |
| | | 3 | 15.4 | 15.0 | 15.4 |
| | | 6 | 15.1 | 15.2 | 14.8 |
| Relative binding activity (direct ELISA, %) | | 0 | 123 | 96 | 85 |
| | | 3 | N/A | N/A | N/A |
| | | 6 | 104 | 104 | 107 |

| | | | | | |
|---|---|---|---|---|---|
| Note: N/A denotes no investigation designed. | | | | | |

### (4) Illumination test

The results of illumination test for formula screening are detailed in Table 10. After illumination at 600±50 Lux for 5 days, formulas 1, 2 and 4 were qualified in appearance and visible particles; protein content (UV method), turbidity (OD_{350 nm} method), purity (SEC-HPLC and non-reduced CE-SDS), charge variants (iCIEF) and relative binding activity (direct ELISA) remained unchanged.

**Table 10. Results of illumination test for formula screening**

| Test items | | Time (day) | Sample name | | |
|---|---|---|---|---|---|
| | | | Formula 1 | Formula 2 | Formula 4 |
| Protein content (UV method, mg/mL) | | 0 | 20.2 | 19.7 | 20.9 |
| | | 3 | 19.4 | 19.3 | 21.1 |
| | | 5 | 19.6 | 19.2 | 20.9 |
| Turbidity (OD_{350 nm} method) | | 0 | 0.076 | 0.074 | 0.080 |
| | | 3 | 0.078 | 0.077 | 0.078 |
| | | 5 | 0.080 | 0.077 | 0.081 |
| Purity (%) | SEC-HPLC | 0 | 98.1 | 98.1 | 98.1 |
| | | 3 | 98.1 | 98.0 | 98.0 |
| | | 5 | 98.1 | 97.9 | 97.9 |
| | Non-reduced CE-SDS | 0 | 98.7 | 98.8 | 98.9 |
| | | 3 | 98.6 | 99.0 | 99.0 |
| | | 5 | 98.4 | 99.0 | 98.9 |
| Charge variants (iCIEF, %) | Acidic component | 0 | 15.7 | 16.1 | 16.7 |
| | | 3 | 17.5 | 17.5 | 17.8 |
| | | 5 | 17.6 | 17.6 | 18.3 |
| | Principal component | 0 | 69.0 | 68.3 | 68.0 |
| | | 3 | 68.1 | 67.6 | 67.4 |
| | | 5 | 67.9 | 67.9 | 67.3 |
| | Basic component | 0 | 15.3 | 15.6 | 15.3 |
| | | 3 | 14.4 | 14.9 | 14.8 |
| | | 5 | 14.5 | 14.5 | 14.4 |
| Relative binding activity (direct ELISA, %) | | 0 | 123 | 96 | 85 |
| | | 3 | N/A | N/A | N/A |
| | | 5 | 73 | 87 | 96 |

| | | | | | |
|---|---|---|---|---|---|
| Note: N/A denotes no investigation designed. | | | | | |

### Conclusion

The results of high-temperature stress tests on formulas 1-7 show that excipients sucrose, arginine, trehalose and sorbitol can be used as the stabilizer of the anti-LAG-3 antibody, wherein excipients sucrose and arginine are superior to trehalose and sorbitol, and can effectively suppress the increase of acidic component and the decrease of principal component.

The results of the shaking test, the freezing-thawing test and the illumination test performed on formulas 1, 2 and 4 suggest that all 3 formulas have good stability, with no significant difference between each other. As such, addition of disodium edetate or methionine has little effect on the stability of the product. For simplification and safety, edetate disodium or methionine will not be added to the formulation.

### Example 4. Formula Screening Test (II)

### 4.1 Procedures

A total of 3 formulas are designed, as detailed in Table 11.Buffers of the formulas were prepared, and the solvent of anti-LAG-3 antibody ADI-31853 was replaced by the buffers via ultrafiltration. After buffer exchange, proteins of the formulas were diluted to about 20 mg/mL and polysorbate-80 was added. The solutions were filtered and filled into vials. The vials were stopped and capped, and were subjected to stability tests, including high-temperature stress test, shaking test and freezing-thawing test. The indexes included appearance, visible particles, protein content (UV method), turbidity (OD_{350 nm} method), purity (SEC-HPLC, non-reduced CE-SDS and reduced CE-SDS), charge variants (iCIEF) and relative binding activity (direct ELISA).

**Table 11. Formulas for screening test (II)**

| No. | Formulas |
|---|---|
| Formula 8 | 5.88 mg/mL sodium citrate (dihydrate), 21.07 mg/mL arginine hydrochloride, 0.70 mg/mL polysorbate-80, pH 6.0 |
| Formula 9 | 5.88 mg/mL sodium citrate (dihydrate), 50.00 mg/mL sucrose, 21.07 mg/mL arginine hydrochloride, 0.70 mg/mL polysorbate-80, pH 6.0 |
| Formula 10 | 5.88 mg/mL sodium citrate (dihydrate), 50.00 mg/mL sucrose, 0.70 mg/mL polysorbate-80, pH 6.0 |

The detailed test conditions and sampling schedule are shown in Table 12.

**Table 12. Test conditions and sampling schedule**

| Test name | Test scheme and sampling time points |
|---|---|
| High temperature stress test | The solutions were let standing at 40±2 °C, and samples were collected at day 0, week 1, week 2 and month 1. |
| Shaking test | 650 rpm, 25±2 °C, away from light; samples were collected on days 0, 1 and 3 |
| Freezing-thawing test | A cycle consisted of a freezing at -20±2 °C for 1 day and a thawing at 5±3 °C; samples were collected after cycles 0, 3 and 6. |

### 4.2. Criteria

See Section 2.2 and Table 3 above.

### 4.3. Results

### (1) High temperature stress test

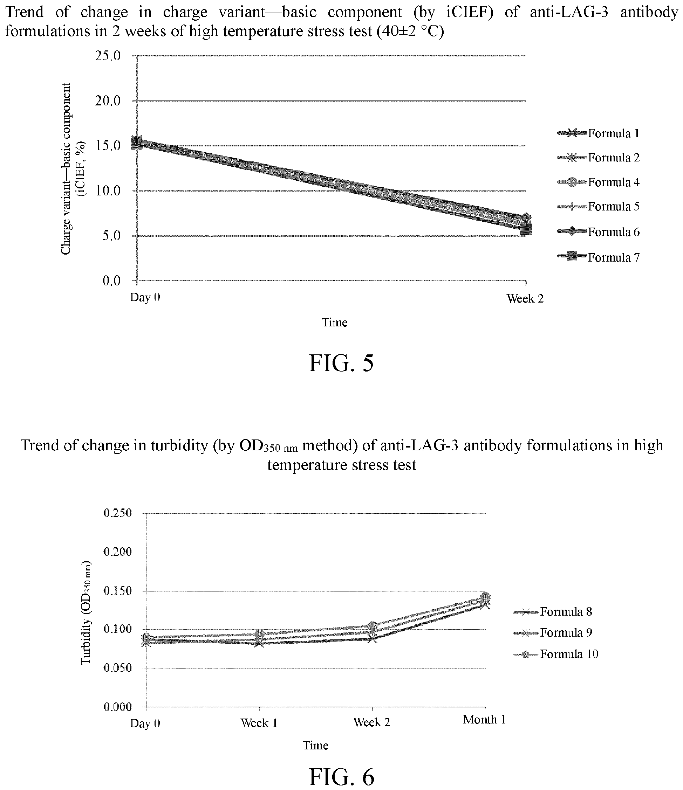

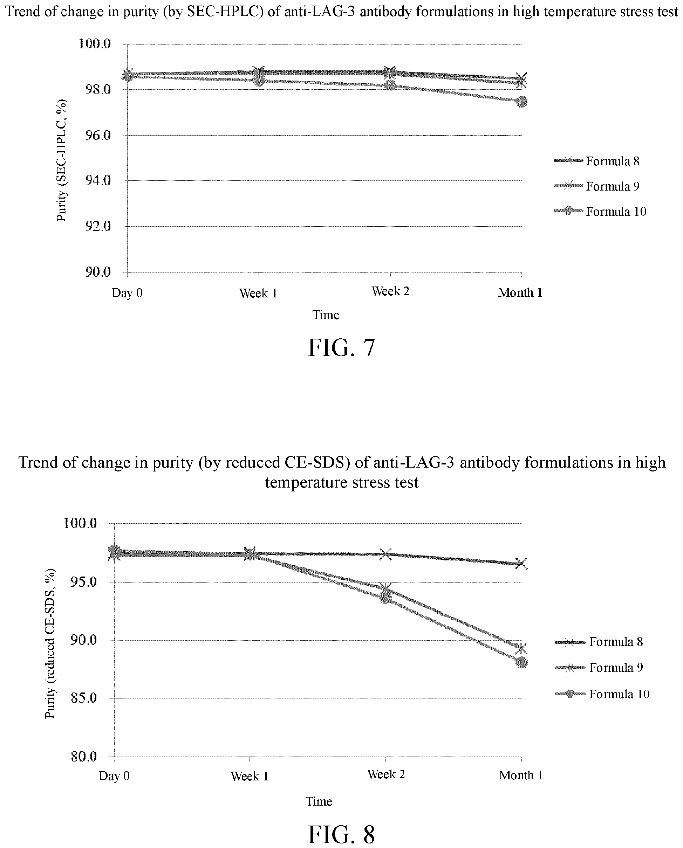

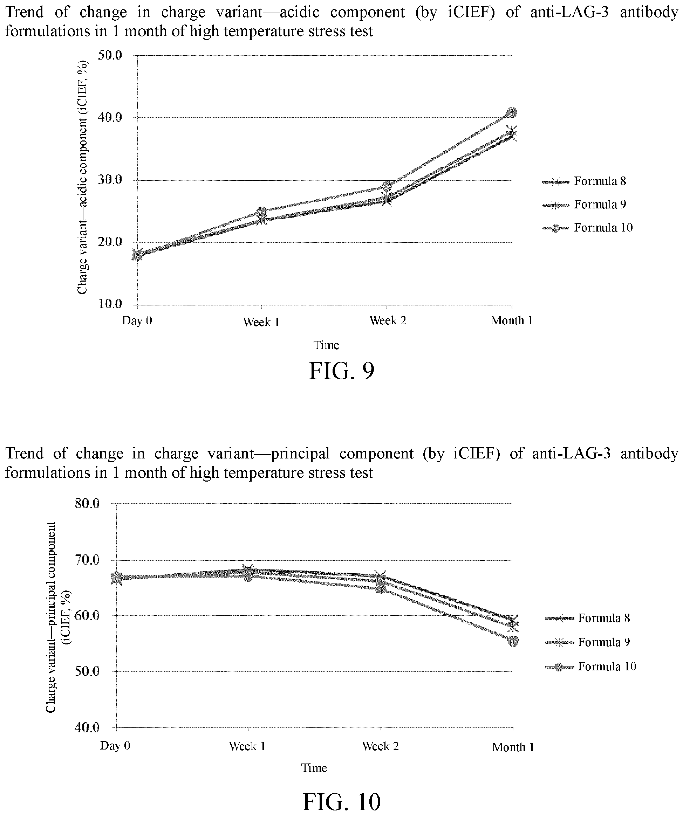

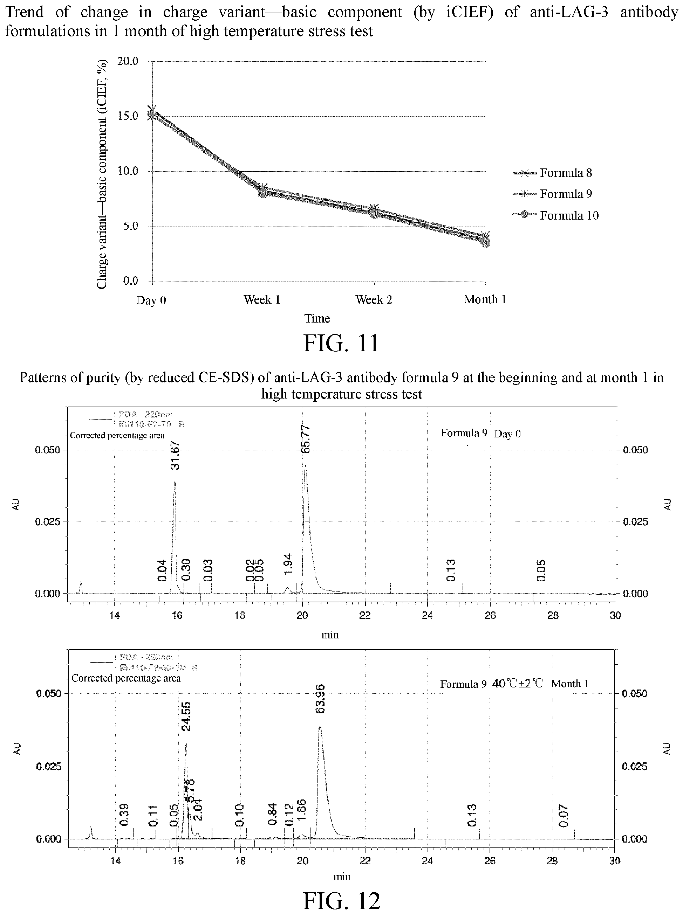

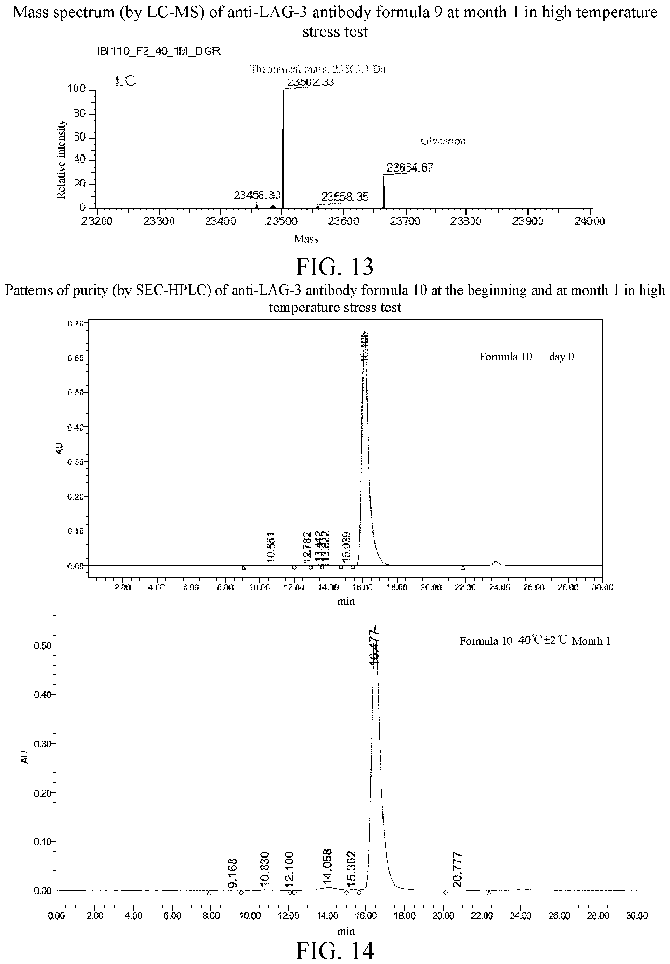

The results of high temperature stress test are detailed in Table 13 and FIGs. 6-14. After standing at 40±2 °C for 1 week, the acidic components (iCIEF) in formulas 8, 9 and 10 increased, while the basic components decreased. After standing at 40±2 °C for 2 weeks, formulas 9 and 10 showed a reduction in purity (reduced CE-SDS) which was caused by glycation as suggested by mass spectrometry, and formula 10 showed a principal component reduction. After standing at 40±2 °C for 1 month, all the formulas were qualified in aspects of appearance and visible particle; the protein content (UV method), the purity (non-reduced CE-SDS) and the relative binding activity (direct ELISA) satisfied the criteria; the purity (reduced CE-SDS and SEC-HPLC) of formula 8 and the purity (SEC-HPLC) of formula 9 remained unchanged; turbidity increased for formulas 8, 9 and 10; principal components decreased for formulas 8, 9 and 10, wherein the decrease of formula 8 was relatively smaller; the purity (SEC-HPLC) decreased for formula 10, mainly attributed to the increase of aggregates.

**Table 13. Results of high temperature stress test for formulation screening (II) (40±2 °C)**

| Test items | | Sampling time points | Sample name | | |
|---|---|---|---|---|---|
| | | | Formula 8 | Formula 9 | Formula 10 |
| Protein content (UV method, mg/mL) | | Day 0 | 21.1 | 20.5 | 20.9 |
| | | Week 1 | 20.8 | 20.3 | 20.3 |
| | | Week 2 | 20.7 | 20.3 | 20.2 |
| | | Month 1 | 20.6 | 19.9 | 20.3 |
| Turbidity (OD_{350 nm} | | Day 0 | 0.087 | 0.083 | 0.090 |
| | | Week 1 | 0.082 | 0.087 | 0.094 |
| | | Week 2 | 0.088 | 0.097 | 0.105 |
| | | Month 1 | 0.132 | 0.138 | 0.142 |
| Purity (%) | SEC-HPLC | Day0 | 98.7 | 98.7 | 98.6 |
| | | Weekl | 98.8 | 98.7 | 98.4 |
| | | Week2 | 98.8 | 98.7 | 98.2 |
| | | Month 1 | 98.5 | 98.3 | 97.5 |
| | Non-reduced CE-SDS | Day0 | 98.4 | 98.3 | 98.6 |
| | | Weekl | 98.0 | 98.1 | 98.3 |
| | | Week2 | 97.7 | 97.9 | 98.6 |
| | | Month 1 | 97.3 | 97.5 | 98.0 |
| | Reduced CE-SDS | Day0 | 97.5 | 97.3 | 97.7 |
| | | Weekl | 97.5 | 97.3 | 97.4 |
| | | Week2 | 97.4 | 94.4 | 93.6 |
| | | Month 1 | 96.6 | 89.3 | 88.1 |
| Charge variants (iCIEF, %) | Acidic component | Day0 | 17.9 | 18.2 | 17.9 |
| | | Weekl | 23.5 | 23.6 | 25.0 |
| | | Week2 | 26.6 | 27.2 | 29.0 |
| | | Month 1 | 37.0 | 37.9 | 40.9 |
| | Principal component | Day0 | 66.5 | 66.7 | 67.0 |
| | | Weekl | 68.3 | 67.9 | 67.1 |
| | | Week2 | 67.1 | 66.2 | 64.9 |
| | | Month 1 | 59.2 | 58.0 | 55.6 |
| | Basic component | Day0 | 15.6 | 15.1 | 15.2 |
| | | Weekl | 8.2 | 8.5 | 8.0 |
| | | Week2 | 6.3 | 6.6 | 6.1 |
| | | Month 1 | 3.8 | 4.1 | 3.5 |
| Relative binding activity (direct ELISA, %) | | Day 0 | 84 | 84 | 85 |
| | | Week 1 | 92 | 86 | 80 |
| | | Week 2 | 101 | 85 | 79 |
| | | Month 1 | 79 | 74 | 88 |

### (2) Shaking test

The results of shaking test for formula screening (II) are detailed in Table 14. After shaking at 650 rpm for 3 days, formulas 8, 9 and 10 were qualified in aspects of appearance and visible particles; protein content (UV method), turbidity (OD_{350 nm} method), purity (non-reduced CE-SDS, reduced CE-SDS and SEC-HPLC), charge variants (iCIEF) and relative binding activity (direct ELISA) remained unchanged.

**Table 14. Results of shaking test for formula screening (II)**

| Test items | Time (day) | Sample name | | | |
|---|---|---|---|---|---|
| | | Formula 8 | Formula 9 | Formula 10 | |
| Protein content (UV method, mg/mL) | 0 | 21.1 | 20.5 | 20.9 | |
| | 1 | 21.4 | 20.5 | 20.9 | |
| | 3 | 21.6 | 20.4 | 20.9 | |
| Turbidity (OD_{350 nm} method) | 0 | 0.087 | 0.083 | 0.090 | |
| | 1 | 0.086 | 0.082 | 0.091 | |

| Test items | | Time (day) | Sample name | | |
|---|---|---|---|---|---|
| | | | Formula 8 | Formula 9 | Formula 10 |
| | | 3 | 0.085 | 0.084 | 0.104 |
| Purity (%) | SEC-HPLC | 0 | 98.7 | 98.7 | 98.6 |
| | | 1 | 98.7 | 98.8 | 98.5 |
| | | 3 | 98.8 | 98.7 | 98.5 |
| | Non-reduced CE-SDS | 0 | 98.4 | 98.3 | 98.6 |
| | | 1 | 98.2 | 98.2 | 98.5 |
| | | 3 | 98.3 | 98.3 | 98.5 |
| | Reduced CE-SDS | 0 | 97.5 | 97.3 | 97.7 |
| | | 1 | 97.4 | 97.4 | 97.6 |
| | | 3 | 97.4 | 97.3 | 97.6 |
| Charge variants (iCIEF, %) | Acidic component | 0 | 17.9 | 18.2 | 17.9 |
| | | 1 | 17.7 | 18.4 | 17.7 |
| | | 3 | 18.6 | 18.4 | 18.4 |
| | Principal component | 0 | 66.5 | 66.7 | 67.0 |
| | | 1 | 67.2 | 66.6 | 67.4 |
| | | 3 | 67.3 | 66.9 | 67.5 |
| | Basic component | 0 | 15.6 | 15.1 | 15.2 |
| | | 1 | 15.2 | 15.0 | 14.9 |
| | | 3 | 14.2 | 14.6 | 14.1 |
| Relative binding activity (direct ELISA, %) | | 0 | 84 | 84 | 85 |
| | | 1 | N/A | N/A | N/A |
| | | 3 | 93 | 96 | 97 |

| | | | | | |
|---|---|---|---|---|---|
| Note: N/A denotes no investigation designed. | | | | | |

### (3) Freezing-thawing test

The results of freezing-thawing test for formula screening (II) are detailed in Table 15. After 6 freezing/thawing cycles, formulas 8, 9 and 10 were qualified in appearance and visible particles; protein content (UV method), turbidity (OD_{350 nm} method), purity (non-reduced CE-SDS, reduced CE-SDS and SEC-HPLC), charge variants (iCIEF) and relative binding activity (direct ELISA) remained unchanged.

**Table 15. Results of freezing-thawing test for formula screening (II)**

| Test items | | Number of freezing/thaw ing cycles | Sample name | | |
|---|---|---|---|---|---|
| | | | Formula 8 | Formula 9 | Formula 10 |
| Protein content (UV method, mg/mL) | | 0 | 21.1 | 20.5 | 20.9 |
| | | 3 | 21.4 | 20.1 | 21.2 |
| | | 6 | 21.1 | 20.6 | 20.6 |
| Turbidity (OD_{350 nm} method) | | 0 | 0.087 | 0.083 | 0.090 |
| | | 3 | 0.086 | 0.079 | 0.091 |
| | | 6 | 0.082 | 0.079 | 0.088 |
| Purity (%) | SEC-HPLC | 0 | 98.7 | 98.7 | 98.6 |
| | | 3 | 98.7 | 98.7 | 98.6 |
| | | 6 | 98.8 | 98.8 | 98.7 |
| | Non-reduced CE-SDS | 0 | 98.4 | 98.3 | 98.6 |
| | | 3 | 98.3 | 98.3 | 98.5 |
| | | 6 | 97.2 | 97.2 | 97.7 |
| | Reduced CE-SDS | 0 | 97.5 | 97.3 | 97.7 |
| | | 3 | 97.6 | 97.2 | 97.6 |
| | | 6 | 97.5 | 97.4 | 97.6 |
| Charge variants (iCIEF, %) | Acidic component | 0 | 17.9 | 18.2 | 17.9 |
| | | 3 | 17.9 | 18.1 | 18.0 |
| | | 6 | 17.9 | 17.8 | 18.2 |
| | Principal component | 0 | 66.5 | 66.7 | 67.0 |
| | | 3 | 66.8 | 66.5 | 66.7 |
| | | 6 | 66.2 | 66.9 | 66.8 |
| | Basic component | 0 | 15.6 | 15.1 | 15.2 |
| | | 3 | 15.3 | 15.4 | 15.3 |
| | | 6 | 15.8 | 15.3 | 15.0 |
| Relative binding activity (direct ELISA, %) | | 0 | 84 | 84 | 85 |
| | | 3 | N/A | N/A | N/A |
| | | 6 | 89 | 93 | 113 |

| | | | | | |
|---|---|---|---|---|---|
| Note: N/A denotes no investigation designed. | | | | | |

### Conclusion

The high temperature stress test results show that for the charge variants (iCIEF), the increase of acid component and the decrease of principal component in formula 8 were relatively smaller; for purity (SEC-HPLC), the formulas 8 and 9 are more advantageous and can effectively suppress the increase of protein aggregates at a high temperature of 40 °C; for purity (reduced CE-SDS), the formula 8 is more advantageous since glycation was not observed at a high temperature of 40 °C. Thus, the most preferred formula was determined as: a recombinant fully human monoclonal anti-lymphocyte activation gene 3 (LAG-3) antibody at about 20 mg/mL, sodium citrate (dihydrate) at about 5.88 mg/mL, arginine hydrochloride at about 21.07 mg/mL and polysorbate 80 at about 0.70 mg/mL, pH 6.0.

The exemplary embodiments of the present invention have been described above. It should be understood by those skilled in the art that these contents are merely exemplary, and various other replacements, adaptations and modifications can be made within the scope of the present invention. Accordingly, the present invention is not limited to the specific embodiments listed herein.

## Claims

1. A liquid antibody formulation at about pH 5.5-7.5, e.g., at about pH 6.0, comprising
(i) an anti-LAG-3 antibody;
(ii) a buffer,
(iii) a stabilizer, and
(iv) a surfactant,
wherein the anti-LAG-3 antibody comprises:
- a heavy chain VH CDR1 of GSIYSESYYWG (SEQ ID NO: 1);
- a heavy chain VH CDR2 of SIVYSGYTYYNPSLKS (SEQ ID NO: 2);
- a heavy chain VH CDR3 of ARVRTWDAAFDI (SEQ ID NO: 3);
- a light chain VL CDR1 of QASQDISNYLN (SEQ ID NO: 4);
- a light chain VL CDR2 of DASNLET (SEQ ID NO: 5); and
- a light chain VL CDR3 of QQVLELPPWT (SEQ ID NO: 6).

2. The liquid antibody formulation according to claim 1, wherein the concentration of the anti-LAG-3 antibody in the liquid antibody formulation is about 1-100 mg/mL, preferably about 5-50 mg/mL, e.g., about 5, 10, 15, 20, 25, 30, 35, 40, 45 or 50 mg/mL.

3. The liquid antibody formulation according to claim 1 or 2, wherein the buffer in the liquid antibody formulation is selected from a citrate, a citrate solvate (e.g., citrate hydrate) and a combination thereof, e.g., sodium citrate, sodium citrate dihydrate and a combination thereof; preferably, the concentration of the buffer is about 1-10 mg/mL, preferably about 4-8 mg/mL, e.g., about 4, 4.5, 5, 5.5, 6, 6.5, 7, 7.5 or 8 mg/mL.

4. The liquid antibody formulation according to any one of claims 1-3, wherein the stabilizer is selected from sorbitol, sucrose, trehalose, arginine, arginine hydrochloride and any combination thereof, and is preferably sucrose, arginine and/or arginine hydrochloride; the concentration of the stabilizer is preferably about 10-150 mg/mL, and more preferably about 15-100 mg/mL, e.g., about 15, 20, 30, 40, 50, 60, 70, 80, 90 or 100 mg/mL.

5. The liquid antibody formulation according to any one of claims 1-4, wherein the liquid antibody formulation comprises arginine and/or arginine hydrochloride as the stabilizer, and preferably, arginine and/or arginine hydrochloride is present in an amount of about 10-40 mg/mL, preferably about 15-25 mg/mL (e.g., about 15, 16, 17, 18, 19, 20, 21, 22, 23, 24 or 25 mg/mL); and/or the liquid antibody formulation comprises sucrose as the stabilizer, and preferably, sucrose is present in an amount of about 20-100 mg/mL, preferably about 40-90 mg/mL (e.g., about 40, 50, 60, 70, 80 or 90 mg/mL).

6. The liquid antibody formulation according to any one of claims 1-5, wherein the surfactant in the liquid antibody formulation is selected from polysorbate surfactants, preferably polysorbate-80.

7. The liquid antibody formulation according to any one of claims 1-6, wherein the concentration of the surfactant is about 0.1-1 mg/mL, preferably about 0.2-0.8 mg/mL, e.g., about 0.2, 0.3, 0.4, 0.5, 0.6, 0.7 or 0.8 mg/mL.

8. The liquid antibody formulation according to any one of claims 1-7, wherein the liquid formulation may or may not comprise an edetate (e.g., disodium edetate) and/or methionine.

9. The liquid antibody formulation according to any one of claims 1-8, wherein the anti-LAG-3 antibody comprises a heavy chain variable region VH and a light chain variable region VL, wherein the heavy chain variable region comprises a sequence of SEQ ID NO: 7 or a sequence having at least 90%, 95%, 98% or 99% identity thereto, and the light chain variable region comprises a sequence of SEQ ID NO: 8 or a sequence having at least 90%, 95%, 98% or 99% identity thereto.

10. The liquid antibody formulation according to any one of claims 1-9, wherein the anti-LAG-3 antibody is an IgG4 antibody, and preferably comprises a heavy chain sequence of SEQ ID NO: 9 or a sequence having at least 90%, 95%, 98% or 99% identity thereto, and a light chain sequence of SEQ ID NO: 10 or a sequence having at least 90%, 95%, 98% or 99% identity thereto.

11. The liquid antibody formulation according to any one of claims 1-10, wherein the anti-LAG-3 antibody is recombinantly expressed in 293 cells or CHO cells.

12. The liquid antibody formulation according to any one of claims 1-11, wherein the liquid formulation is an injection, preferably for use in subcutaneous injection or intravenous injection; or an infusion, e.g., for use in intravenous infusion.

13. The liquid antibody formulation according to claim 1, wherein the liquid antibody formulation comprises:
(i) the anti-LAG-3 antibody at about 1-100 mg/mL;
(ii) sodium citrate or sodium citrate dihydrate at about 1-10 mg/mL;
(iii) sucrose, arginine and/or arginine hydrochloride at about 10-150 mg/mL; and
(iv) polysorbate 80 at about 0.1-1 mg/mL; wherein
optionally, the liquid formulation does not comprise an edetate (e.g., disodium edetate) and methionine;
wherein the pH of the liquid formulation is about 5.5-7.5, preferably about 6.0-7.0, and more preferably 6.0±0.3, e.g., 6.0; preferably, the pH is adjusted by anhydrous citric acid; or
for example, the liquid antibody formulation comprises:
(i) the anti-LAG-3 antibody at about 10-30 mg/mL;
(ii) sodium citrate or sodium citrate dihydrate at about 2-8 mg/mL;
(iii) arginine and/or arginine hydrochloride at about 10-40 mg/mL, and/or sucrose at about 40-90 mg/mL; and
(iv) polysorbate-80 at about 0.2-0.8 mg/mL; wherein
optionally, the liquid formulation does not comprise an edetate (e.g., disodium edetate) and methionine;
wherein the pH of the liquid formulation is about 5.5-7.5, preferably about 6.0-7.0, and more preferably 6.0±0.3, e.g., 6.0; preferably, the pH is adjusted by anhydrous citric acid; or
the liquid antibody formulation comprises:
(i) the anti-LAG-3 antibody at about 20 mg/mL;
(ii) sodium citrate dihydrate at about 5.88 mg/mL;
(iii) sucrose at about 80 mg/mL; and
(iv) polysorbate 80 at about 0.3 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid; or
the liquid antibody formulation comprises:
(i) the anti-LAG-3 antibody at about 20 mg/mL;
(ii) sodium citrate dihydrate at about 5.88 mg/mL;
(iii) arginine at about 17.42 mg/mL; and
(iv) polysorbate 80 at about 0.3 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid; or
the liquid antibody formulation comprises:
(i) the anti-LAG-3 antibody at about 20 mg/mL;
(ii) sodium citrate dihydrate at about 5.88 mg/mL;
(iii) arginine hydrochloride at about 21.07 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid; or
the liquid antibody formulation comprises:
(i) the anti-LAG-3 antibody at about 20 mg/mL;
(ii) sodium citrate dihydrate at about 5.88 mg/mL;
(iii) sucrose at about 50 mg/mL and arginine hydrochloride at about 21.07 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid; or
the liquid antibody formulation comprises:
(i) the anti-LAG-3 antibody at about 20 mg/mL;
(ii) sodium citrate dihydrate at about 5.88 mg/mL;
(iii) sucrose at about 50 mg/mL; and
(iv) polysorbate 80 at about 0.7 mg/mL;
wherein the pH of the liquid formulation is about 5.5-7.5, preferably about 6.0-7.0, and more preferably 6.0±0.3, e.g., 6.0; preferably, the pH is adjusted by anhydrous citric acid.

14. The liquid antibody formulation according to any one of claims 1-13, wherein the formulation is stable after storage, e.g., at 2-8 °C for at least 24 months, at room temperature for at least 3 months, or at 40±2 °C for 1 month, and preferably, has one or more of the following characteristics:
(i) a purity greater than 90%, preferably greater than 95%, 96%, 97%, 98% or 99%, as measured by SEC-HPLC;
(ii) a purity greater than 90%, preferably greater than 95%, 96%, 97%, 98% or 99%, as measured by reduced or non-reduced CE-SDS;
(iii) changes ≤ 2% in components of the anti-LAG-3 antibody (i.e., principal component, acidic component and basic component) in the formulation relative to an initial value on day 0 of storage as measured by iCIEF; and
(iv) a relative binding activity of the anti-LAG-3 antibody in the formulation of 70-130%, e.g., 70%, 80%, 90%, 100%, 110%, 120% or 130%, relative to an initial value on day 0 of storage as measured by ELISA.

15. A solid antibody formulation obtained by solidifying the liquid antibody formulation according to any one of claims 1-14, wherein the solid formulation is, e.g., in the form of lyophilized powder for injection.

16. A delivery device, comprising the liquid antibody formulation according to any one of claims 1-14 or the solid antibody formulation according to claim 15.

17. A pre-filled syringe, comprising the liquid antibody formulation according to any one of claims 1-14 or the solid antibody formulation according to claim 15 for use in intravenous or intramuscular injection.

18. Use of the liquid antibody formulation according to any one of claims 1-14 or the solid antibody formulation according to claim 15 in preparing a medicament for treating, preventing or delaying an LAG-3-expressing cancer (particularly a metastatic cancer), an immune-related disease and a T cell dysfunctional disease.
